(19) Europäisches Patentamt
European Patent Office
Office européen des brevets

(11) EP 2 067 490 A1

# (12) EUROPEAN PATENT APPLICATION

(43) Date of publication:
**10.06.2009 Bulletin 2009/24**

(51) Int Cl.:
***A61K 49/08*** *(2006.01)*

(21) Application number: **07122630.2**

(22) Date of filing: **07.12.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Koninklijke Philips Electronics N.V.**
**5621 BA Eindhoven (NL)**

(72) Inventor: **The designation of the inventor has not yet been filed**

(74) Representative: **Van Velzen, Maaike Mathilde**
**Philips**
**Intellectual Property & Standards**
**P.O. Box 220**
**5600 AE Eindhoven (NL)**

## (54) Chelating agent derivatives and compositions comprising the same for use in imaging methods

(57) Chelating agent derivatives and methods to produce and use them are disclosed. The chelating agent derivatives comprise one or more chelating moieties attached either directly or via a linker to a phospholipid moiety. The chelating agent derivatives of the present invention are suitable to be incorporated in a great number of copies into a lipophilic nanoparticle or microparticle. They are advantageously used in imaging methods such as e.g. MRI.

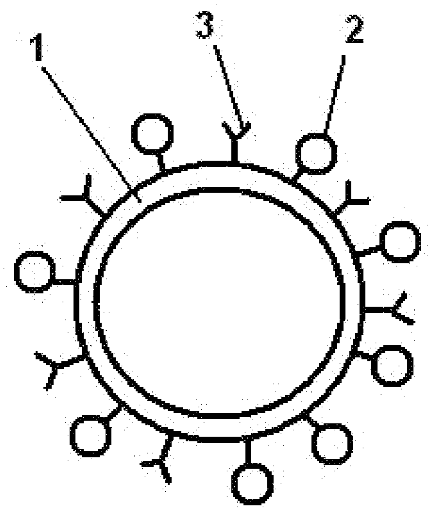


FIG. 1



Printed by Jouve, 75001 PARIS (FR)

## Description

BACKGROUND TO THE INVENTION

The present invention relates to chelating agents derivatives useful in molecular imaging as well as to methods of making and using the same.

The use of chelates as contrast agents in imaging techniques is well known. Molecular Imaging (MI) is a very promising new field in medicine which, for instance, aims at the in-vivo characterization of biological pathways. This should enable the detection of diseases at a much earlier stage than at present. Different technologies will play a role in MI as they all have advantages. For instance, nuclear imaging techniques (e.g. Positron Emission Tomography (PET) or Single Photon Emission Computed Tomography (SPECT)) are more sensitive than Magnetic Resonance Imaging (MRI) but do not permit a spatial resolution as high as MRI. One way to overcome the sensitivity problem of MRI, is to provide to the location of interest lipophilic micro- or nano-particles such as liposomes or perfluorocarbon emulsions, preferably comprising contrast-enhancing moieties.

US2006/0008417 discloses a chelating agent derivative of the following formula:

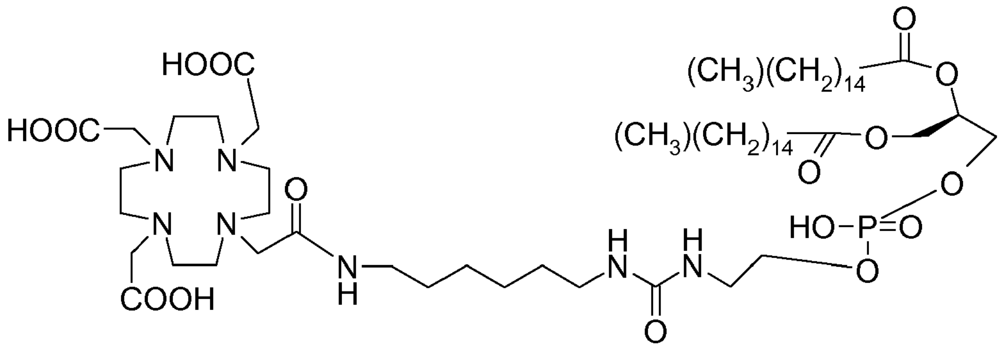

comprising a 1,4,7,10-tetraaza-cyclododecane-N,N',N'',N'''-tetraacetic acid (DOTA) group linked via a linker to a phosphoethanolamine lipid moiety. This chelating agent is an example of derivative that can be incorporated into a lipophilic micro- or nano-emulsion. There is a constant need in the fast evolving field of imaging for new chelating agent derivatives.

It is an object of the present invention to provide alternative chelating agent derivatives and methods of making and using the same. An advantage of certain embodiments of the present invention is to provide chelating agent derivatives for incorporation in a great number into a lipophilic nano- or microparticle while simultaneously displaying good relaxivity. Another advantage of certain embodiments of the present invention is that the synthesis of these novel chelating agent derivatives is simple.

The above objective is accomplished by methods and derivatives according to the present invention.

SUMMARY OF THE INVENTION

In a first embodiment, the present invention relates to a chelating agent derivative represented by the structural formula:

$$(Ch\text{-}CO)_mNR''\text{-}L_1\text{-}CH_2\text{-}CH_2\text{-}O\text{-}P(=O)(X)OCH_2CH(OCOR_2)(CH_2OCOR_3) \qquad (I)$$

wherein

- $R_2$ and $R_3$ are independently selected from the group comprising, or consisting of, $C_{12}$-$C_{22}$ alkyl and $C_{12}$-$C_{22}$ alkenyl,
- Ch is a chelating moiety capable of chelating a paramagnetic metal ion or a radionucleide metal,
- m is an integer ranging from 1 to 10,

wherein

- when m=1, $L_1$ is a linker selected from the group consisting of a single bond, oligopeptides, polypeptides, oligoethylene glycol, polyethylene glycol or $L_2$CONR' wherein $L_2$ is a hydrocarbon chains having from 2 to 40 carbon atoms, said hydrocarbon chains optionally comprising one or more oxygen atoms in the main chain and/or being further

optionally substituted with one or more substituents independently selected from the group consisting of $C_{1-4}$ alkyl and $C_{1-4}$ alkoxy and optionally comprising at least one primary amino group,

- when m is 2 to 10, $L_1$ is a linker selected from the group consisting of
- (a) oligopeptides or polypeptides comprising at least m-1 pending groups, m-1 of said pending groups being each directly coupled to a Ch-CO- group, and
- (b) $L_2$CONR', wherein $L_2$ is a hydrocarbon chain having from 2 to 40 carbon atoms, said hydrocarbon chain comprising at least m-1 pending groups, m-1 of said pending groups being each directly coupled to a Ch-CO- group, said hydrocarbon chain optionally further comprising one or more oxo groups and/or heteroatoms independently selected from oxygen and nitrogen in the main chain,
- R' and R" are independently hydrogen or a $C_1$-$C_4$ alkyl chain,
- X is OH or $O^-M^+$, and
- $M^+$ is a counter-ion,

or a salt or a stereoisomeric form thereof.

Particular and preferred aspects of the invention are set out in the accompanying independent and dependent claims. Features from the dependent claims may be combined with features of the independent claims and with features of other dependent claims as appropriate and not merely as explicitly set out in the claims.

As an advantageous feature of certain embodiments of the present invention, the chelating moiety Ch may comprise at least two nitrogens spaced by alkylene groups and to which carboxylic acid-bearing moieties are coupled.

As an advantageous feature of certain embodiments of the present invention, Ch may be a chelating moiety derived from one of ethylenediamine tetraacetic acid (EDTA), diethylenetriamine-N,N,N',N",N"-pentaacetate (DTPA), 1,4,10,13-tetraoxa-7,16-diazacyclooctadecane-7 (ODDA) 16-diacetate, *N*-2-(azol-1-yl)ethyliminodiacetic acid, 1,4,7,10-tetraaza-cyclododecane-N,N',N'',N'''-tetraacetic acid (DOTA), 1,7,13-triaza-4,10,16-trioxacyclooctadecane-N,N',N"-triacetate (TTTA), tetraethyleneglycol-1,5,9-triazacyclododecane-N,N',N",-tris(methylenephosphonic acid) (DOTRP) or 1,4,7,10-tetraazacyclododecane-$\alpha$,$\alpha$',$\alpha$'',$\alpha$'''-tetramethyl-N,N',N'',N'''-tetraacetic acid (DOTMA). The use of these chelating agents is advantageous because they have good chelating properties, especially with respect to a wide range of paramagnetic metal ions and radionucleide metals.

As another advantageous feature of certain embodiments of the present invention, m may be 2 to 10 and $L_1$ may be an oligopeptide or a polypeptide comprising at least m-1 lysine residues.

As another advantageous feature of certain embodiments of the present invention, m may be 2 to 10 and $L_1$ may be an oligopeptide or a polypeptide comprising at least m-1 lysine residues, wherein m-1 of said lysine residues are each directly coupled to a (Ch-CO)- group. This is advantageous because the presence of more than one chelating moieties on a phospholipid permits for instance an increase of the contrast in MRI experiments. The synthesis of such derivatives represents both a versatile and efficient way to attach more than one chelating moiety to a phospholipid.

As another advantageous feature of certain embodiments of the present invention, when m is from 2 to 10, $L_1$ may be a oligolysine or a polylysine comprising lysine residues, wherein m-1 of said lysine residues are each directly coupled to a (Ch-CO)- group. This represent both an elegant and efficient way to attach more than one chelating moiety to a phospholipid since it does not require too much synthetic effort.

As an advantageous feature of certain embodiments of the present invention, $L_2$ may be an oligo- or a polyethyleneglycol chain. This is advantageous because oligo- or a polyethyleneglycol chains are relatively biocompatible. According to the present invention, an oligo-chain refers to the presence of at most 10 repeating ethyleneglycol moieties, and a polyethyleneglycol chain refers to the presence of more than 10 repeating ethyleneglycol moieties in the chain.

As an advantageous feature of certain embodiments of the present invention, the chelating agent derivative may be represented by the following structural formula :

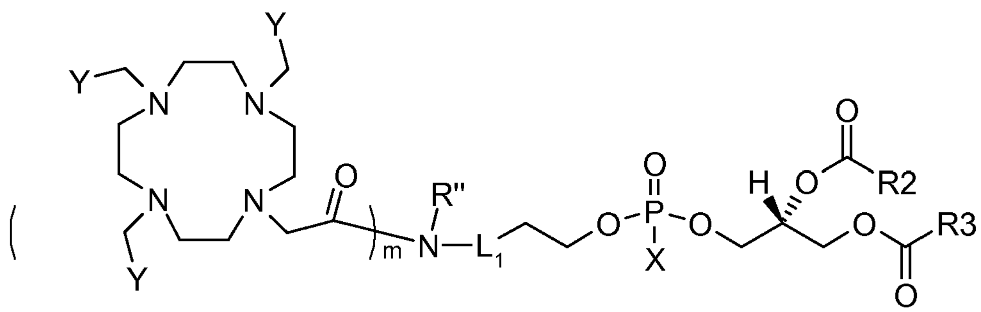

(III)

wherein:

- Y is selected from the group consisting of a carboxylic acid group, a carboxylate ion or salt, or a carboxylic acid methyl ester, methoxymethyl ester, methylthiomethyl ester, tetrahydropyranyl ester, benzyloxymethyl ester, phenacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester, 2-(9,10-dioxo)anthrylmethyl ester, piperonyl ester, trimethylsilyl ester, t-butyldimethylsilyl ester, S-t-butyl ester, and 2-alkyl-1,3-oxazolines,
- X is OH or $O^-M^+$,
- $M^+$ is a counter-ion,

m is an integer ranging from 1 to 10,
wherein

- when m=1, $L_1$ is a linker selected from the group consisting of a single bond, oligopeptides, polypeptides, oligoethylene glycol, polyethylene glycol or $L_2$CONR' wherein $L_2$ is a hydrocarbon chain having from 2 to 40 carbon atoms, said hydrocarbon chain optionally comprising one or more oxygen atoms in the main chain and/or being further optionally substituted with one or more substituents independently selected from the group consisting of $C_{1-4}$ alkyl and $C_{1-4}$ alkoxy and optionally comprising at least one primary amino group,
- when m is 2 to 10, $L_1$ is a linker selected from the group consisting of
- (a) oligopeptides or polypeptides comprising at least m-1 pending groups, m-1 of said pending groups being each directly coupled to a Ch-CO- group, and
- (b) $L_2$CONR', wherein $L_2$ is a hydrocarbon chain having from 2 to 40 carbon atoms, said hydrocarbon chain comprising at least m-1 pending groups, m-1 of said pending groups being each directly coupled to a Ch-CO- group, said hydrocarbon chain optionally further comprising one or more oxo groups and/or heteroatoms independently selected from oxygen and nitrogen in the main chain,
- R' and R" are independently hydrogen or a $C_1$-$C_4$alkyl chain,
- X is OH or $O^-M^+$, and

$M^+$ is a counter-ion, a salt or a stereoisomeric form thereof. This embodiment of the invention is advantageous because chelating moieties derived from DOTA are particularly good at complexing various metals including e.g. radionuclide metals or metal suitable for optical or
X-ray imaging. Preferably, Y is selected from the group consisting of a carboxylic acid group and carboxylate ions or salts.
As another advantageous feature of certain embodiments of the present invention, the chelating agent derivative may be represented by one of the following structural formulae:

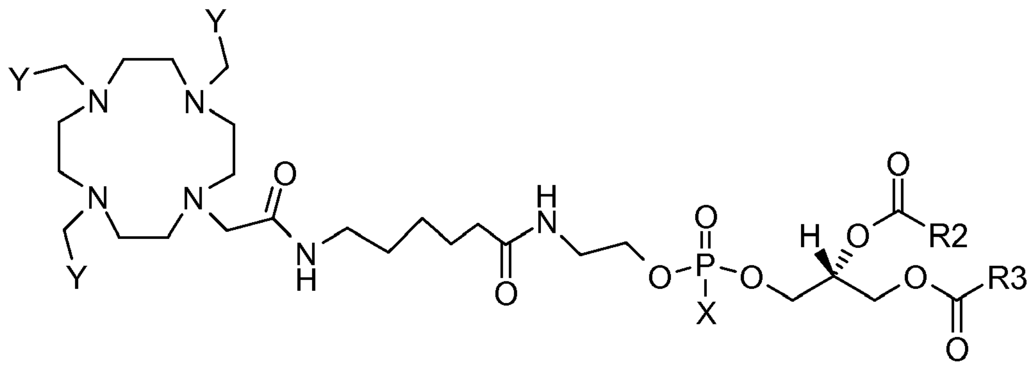

(X)

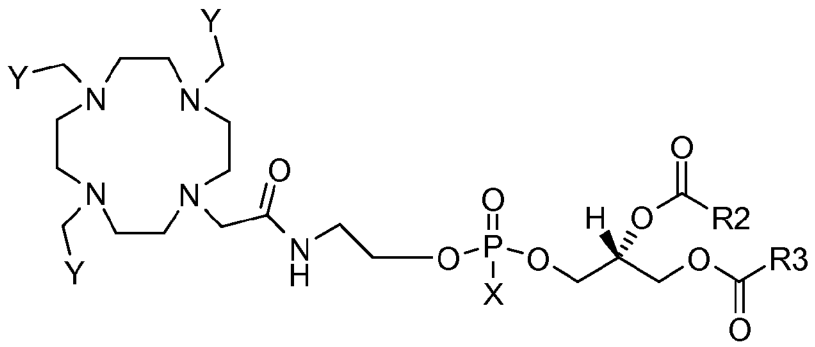

(XI)

wherein:

- X is OH or $O^-M^+$,
- Y is selected from the group consisting of a carboxylic acid group, a carboxylate ion or salt, a carboxylic acid methyl ester, methoxymethyl ester, methylthiomethyl ester, tetrahydropyranyl ester, benzyloxymethyl ester, phenacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester, 2-(9,10-dioxo)anthrylmethyl ester, piperonyl ester, trimethylsilyl ester, t-butyldimethylsilyl esteror S-t-butyl ester, and 2-alkyl-1,3-oxazolines, and
- $R_2$ and $R_3$ are independently selected from the group consisting of $C_{12}$-$C_{22}$ alkyl and $C_{12}$-$C_{22}$ alkenyl.

Those are particularly efficient chelating agent derivatives. As another advantageous feature of certain embodiments of the present invention, the chelating agent derivative may be represented by one of the following structural formulae:

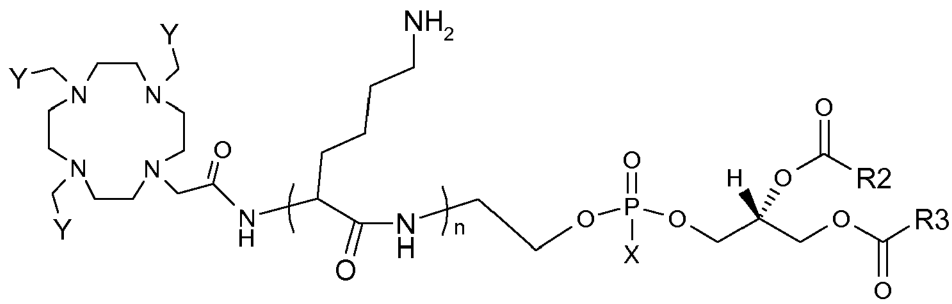

(VI)

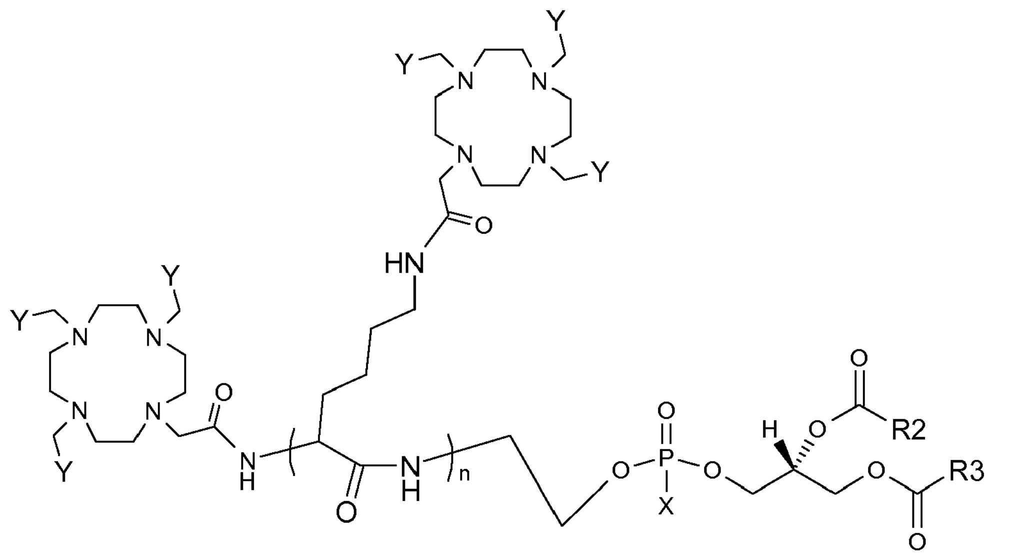

(VII)

wherein:

- X is OH or $O^-M^+$,
- Y is selected from the group consisting of a carboxylic acid group, a carboxylate ion or salt, a carboxylic acid methyl ester, methoxymethyl ester, methylthiomethyl ester, tetrahydropyranyl ester, benzyloxymethyl ester, phenacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester, 2-(9,10-dioxo)anthrylmethyl ester, piperonyl ester, trimethylsilyl ester, t-butyldimethylsilyl ester, or S-t-butyl ester, and 2-alkyl-1,3-oxazolines, and

$R_2$ and $R_3$ are independently selected from the group consisting of $C_{12}$-$C_{22}$ alkyl and $C_{12}$-$C_{22}$ alkenyl, a salt or a stereoisomer thereof. This embodiment of the invention is advantageous because the presence of more than one chelating moieties on a phospholipid permits for instance an increase of the contrast in MRI experiments. The synthesis of such a compounds represents both a versatile and efficient way to attach more than one chelating moiety to a phospholipid.

As another advantageous feature of the present invention, the chelating agent derivative of any of the embodiments above may further include a paramagnetic metal ion or a radionuclide metal chelated with the chelating moiety. This embodiment is advantageous to enable the chelating agent derivative to act as a contrast agent in imaging techniques.

As another advantageous feature of certain embodiments of the present invention, the paramagnetic metal ion may be Gd(3+).

This embodiment is advantageous because Gd(3+) gives very good contrast in MRI for instance.

In another embodiment, the present invention relates to a composition comprising both a lipophilic nanoparticle or microparticle and a chelating agent derivative according to any of the embodiments described above. Such a composition is advantageous because it facilitates the delivery of the chelating agent derivative into an animal or human body to the locus of interest.

As an advantageous feature of certain embodiments of the composition described above, the lipophilic nanoparticle or microparticle further comprises a targeting agent and/or a biologically active agent.

The use of a targeting agent in such an embodiment is advantageous because it permits to have the chelating agent delivered at the location of interest in the animal or human body. The use of a biologically active agent in such an embodiment is advantageous because it permits to perform an imaging method at the precise location where the biologically active agent more specifically has its activity. The type of biologically active agent suitable in such an embodiment of the present invention is not a critical parameter and may be properly selected by the skilled person based on standard knowledge in the art, and taking into account the location of interest in the animal or human body as well as the type of imaging method to be performed.

As an advantageous feature of certain embodiments of the present invention, the targeting agent may be a receptor ligand or an antibody, or a fragment thereof. This embodiment is advantageous because they usually are efficient targeting agents. The type of targeting agent suitable in such an embodiment of the present invention is not a

critical parameter and may be properly selected by the skilled person based on standard knowledge in the art, and taking into account the location of interest in the animal or human body as well as the type of imaging method to be performed.

In another embodiment, the present invention relates to an imaging method for imaging an object including a chelating agent derivative, or a composition comprising the same, according to any of the embodiments described hereinabove, at a location to be imaged, the said method comprising imaging the object with the said chelating agent derivative or composition included therein. Within the present embodiment, the object to be imaged may be an animal or human body, or a portion thereof.

This embodiment of the invention has the advantage, due to the specific features of the chelating agent derivative, to enhance the sensitivity of the imaging method.

As an advantageous feature of this embodiment of the present invention, said imaging method is selected from the group consisting of magnetic resonance imaging, radionuclide imaging, optical imaging, X-ray imaging, or a combination thereof.

In another embodiment, the present invention relates to a method of manufacturing a chelating agent derivative according to any of the embodiments described hereinabove, said method comprising a step of reacting a phosphoethanolamine lipid, or a derivative thereof, with a chelating moiety having at least one carboxylic acid group.

As an optional and advantageous feature of the method of manufacturing according to an embodiment of the present invention, one of the at least one carboxylic acid group of said chelating moiety may be coupled through a linker to said phosphoethanolamine lipid moiety. In such a specific embodiment, said manufacturing method comprises the step of reacting said phosphoethanolamine lipid with said linker to first form a phosphoethanolamine lipid derivative, and then in a second step reacting said phosphoethanolamine lipid derivative with said chelating moiety having at least one carboxylic acid group.

As another advantageous feature of the method of manufacturing according to an embodiment of the present invention, the at least one carboxylic acid group of said chelating moiety may be coupled through a linker to said phosphoethanolamine lipid moiety. In such another specific embodiment, said manufacturing method comprises the step of reacting said chelating moiety with said linker to first form a chelating moiety intermediate, and then in a second step reacting said chelating moiety intermediate with said phosphoethanolamine lipid.

As another advantageous feature of any of the embodiments of the method of manufacturing according to the present invention, the linker may have at least a first terminal amino group reactive with the at least one carboxylic acid group of said chelating moiety, and a second terminal carbonyl or sulfonyl group reactive with the terminal amino group of said phosphoethanolamine lipid.

In another embodiment, the present invention relates to an imaging system comprising:

- a chelating agent derivative, or a composition comprising the same, according to any of the embodiments described hereinabove, and
- an equipment for performing imaging of an object, said imaging comprising the detection of said chelating agent derivative or composition in said object.

Within the present embodiment, the object to be imaged by said equipment may be an animal or human body, or a portion thereof. For instance, when the chelating agent derivative includes a radionuclide metal chelated with the chelating moiety, the equipment comprised within the imaging system of the present invention is an equipment suitable for detecting radioactivity.

As an advantageous feature of certain embodiments of the present invention, the imaging equipment comprised within the imaging system of the present invention may be selected from the group comprising, or consisting of, magnetic resonance imaging equipment, radionuclide imaging equipment, optical imaging equipment, X-ray imaging equipment, or a combination thereof.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

## BRIEF DESCRIPTION OF THE FIGURES

Fig. 1 is a schematic representation of a composition according to an embodiment of the present invention.

Fig. 2 is a synthetic scheme describing the synthesis of Gd(III)DOTA-C6-phospholipids **21a** ($R=C_{15}H_{31}$) and **21b** ($R=C_{17}H_{35}$) in three steps. (a) 1:1 v/v DMF/$CHCl_3$, HBTU, DiPEA; (b) 1:2 v/v TFA/DCM; (c) $Gd(III)(OAc)_3 \cdot 6\ H_2O$, 3:30:67 v/v/v $H_2O$/MeOH/$CHCl_3$

Fig. 3 is a synthetic scheme describing the synthesis of Gd(III)DOTA-C6-phospholipids **18a** ($R=C_{15}H_{31}$) and **18b**

($R=C_{17}H_{35}$) in three steps. (a) 1:1 v/v DMF/$CHCl_3$, HBTU, DiPEA; (b) 1:2 v/v TFA/DCM; (c) Gd(III)$(OAc)_3\cdot 6\ H_2O$, 3:30:67 v/v/v $H_2O$/MeOH/$CHCl_3$.

In the different figures, the same reference signs refer to the same or analogous elements.

DETAILED DESCRIPTION

The present invention will be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes.

Where the term "comprising" is used in the present description and claims, it does not exclude other elements or steps. Where an indefinite or definite article is used when referring to a singular noun e.g. "a" or "an", "the", this includes a plural of that noun unless something else is specifically stated.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

Reference throughout this specification to "one embodiment" or " an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

In a first aspect, the present invention relates to a chelating agent derivative represented by the structural formula (I)

$$(Ch\text{-}CO)nrNR''\text{-}L_1\text{-}CH_2\text{-}CH2\text{-}O\text{-}P(=O)(X)OCH_2CH(OCOR_2)(CH_2OCOR_3) \qquad (I)$$

wherein Ch is a chelating moiety derived from a chelating agent, and wherein each of $R_2$, $R_3$, X, R", m and $L_1$, are as defined hereinabove. In an embodiment of the present invention, the chelating agent from which Ch is derived is a chelating agent comprising at least one carboxylic acid group. In this embodiment of the present invention, one of the carboxylic acid groups of the chelating agent, or its only carboxylic acid group, is not present anymore in the derivative represented by the structural formula (I) since it has been reacted for instance with a phosphoethanolamine derivative represented by the structural formula $HNR''\text{-}L_1\text{-}CH_2\text{-}CH_2\text{-}O\text{-}P(=O)(X)OCH_2CH(OCOR_2)(CH_2OCOR_3)$, or a phosphoethanolamine moiety directly. As a result, in this embodiment of the present invention, the chelating moiety Ch is represented linked to the carbonyl group originating from the carboxylic acid, or from one of the carboxylic acids, originally present in the chelating agent, said carbonyl group being itself linked to $-NR''\text{-}L_1\text{-}CH_2\text{-}CH_2\text{-}O\text{-}P(=O)(X)OCH_2CH(OCOR_2)$

($CH_2OCOR_3$) or to a phosphoethanolamine moiety.

Examples of suitable chelating agents, from which the chelating moiety Ch present in the structural formula (I) can be derived, comprise but are not limited to ethylenediamine tetraacetic acid (EDTA); diethylenetriamine-N,N,N',N'',N''-pentaacetate (DTPA); 1,4,10,13-tetraoxa-7,16-diazacyclo-octadecane-7,16-diacetate (ODDA); N-2-(azol-1-yl)ethyliminodiacetic acid; 1,4,7,10-tetraaza-cyclododecane-N,N',N'',N'''-tetraacetic acid (DOTA), 1,7,13-triaza-4,10,16-trioxacyclooctadecane-N,N',N''-triacetate (TTTA) and α,α',α'',α'''-tetramethyl-1,4,7,10-tetraazacyclododecane-N,N',N'',N'''-tetraacetic acid (DOTMA), and analogues thereof, among others. In some embodiments of the present invention, the chelating moiety Ch can be for instance derived from any of the above mentioned chelating agents.

In a particular embodiment of this invention, the chelating moiety Ch may be derived from DOTA.

In the structural formula (I) above, m is an integer representing the number of chelating moieties Ch in the chelating agent derivative. m can take any value from 1 to 10. In some embodiments, m is 1; in some other embodiments m is any value between 2 and 10. When $L_1$ is not present, i.e. when $L_1$ is a single bond, m is 1. When $L_1$ is not a single bond, it is a linker being represented by the structural formula

$$-L_2CONR'- \quad (II)$$

wherein $L_2$ is as defined hereinabove.

In some embodiments of this invention, $L_2$ may be a saturated aliphatic hydrocarbon chain having from 2 to 40 carbon atoms having a first terminal amino group and a second terminal carbonyl or sulfonyl group, said first terminal amino group being coupled to the at least one carboxylic acid group of said chelating agent, and said second terminal carbonyl or sulfonyl group being coupled to the amino group of said phosphoethanolamine lipid moiety.

In some embodiments of this invention, the number of carbon atoms of $L_2$ may be from 4 to 40, or from 4 to 10, or from 6 to 40. In any of these embodiments, $L_2$ may further comprise one or more heteroatoms independently selected from oxygen and nitrogen in the main chain. In some embodiments of this invention, $L_2$ may be an oligo- or a polyethyleneglycol chain such as defined hereinabove.

In some embodiments of the present invention, one or more chelating moieties Ch may optionally be attached to $L_1$, e.g. attached to pending groups comprised within $L_1$.

$L_1$ may have, in some embodiments of the present invention, one or more pending groups directly coupled to at least a chelating agent. $L_1$ may for instance be an oligopeptide or a polypeptide, both terms being as defined hereinabove, comprising at least m-1 lysine groups, m-1 of said lysine groups being each directly coupled to a Ch-CO- group. Also, $L_1$ may be an oligolysine or a polylysine comprising lysine residues, wherein m-1 of said lysine residues are each directly coupled to a (Ch-CO)- group.

In the above formula, R' and R'' are independently hydrogen or a $C_1$-$C_4$ alkyl chain, X is OH or $O^-M^+$, and $M^+$ is a counter-ion, including but not limited to alkali and alkaline earth metal ions, as well as inorganic salts such as ammonium, and other counter-ions known in the art. Suitable counter-ions include, but are not limited to, $Na^+$, $K^+$, $Ca^{2+}$, $R_3NH^+$ and $R_4N^+$, wherein each R is independently selected from hydrogen and alkyl. In the above formula, $R_2$ and $R_3$ are independently selected from the group consisting of $C_{12}$-$C_{22}$ alkyl and $C_{12}$-$C_{22}$ alkenyl.

In one preferred embodiment, the chelating agent derivative is represented by the following structural formula:

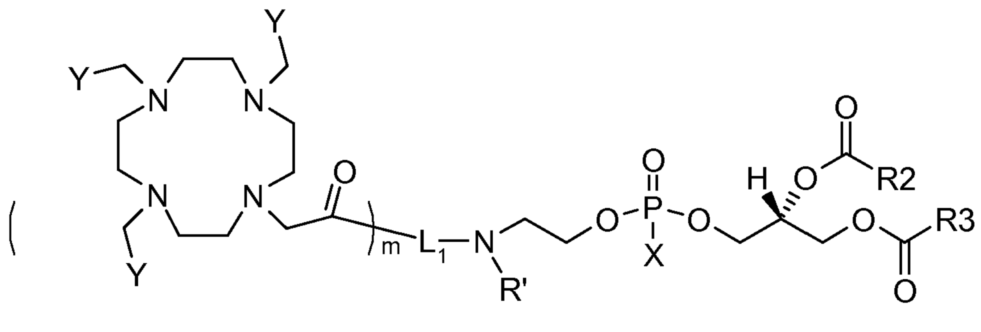

(III)

wherein Y, m, $L_1$, R', X, $R_2$ and $R_3$ are as defined above.

Non-limiting examples of chelating agent derivative according to some embodiments of the present invention are given below. For each of those examples, Y, $R_2$ and $R_3$ are as defined above:

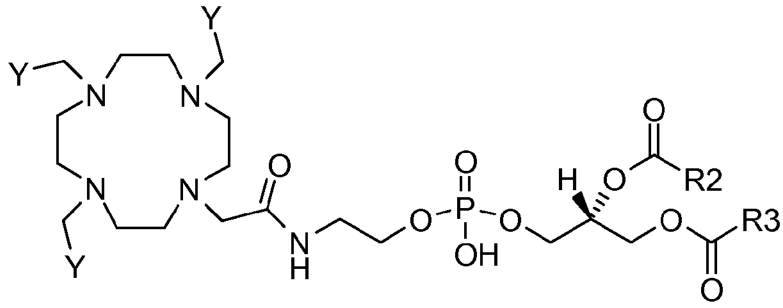

(IV)

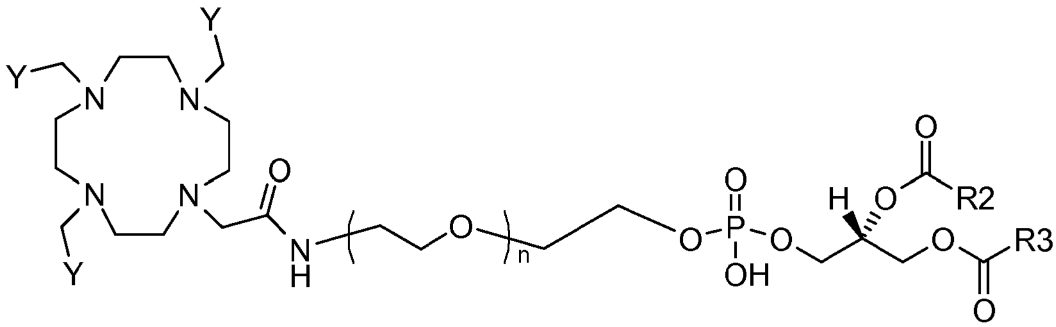

(V)

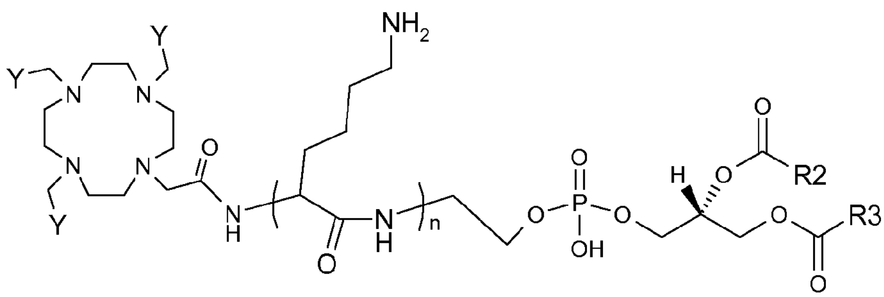

(VI)

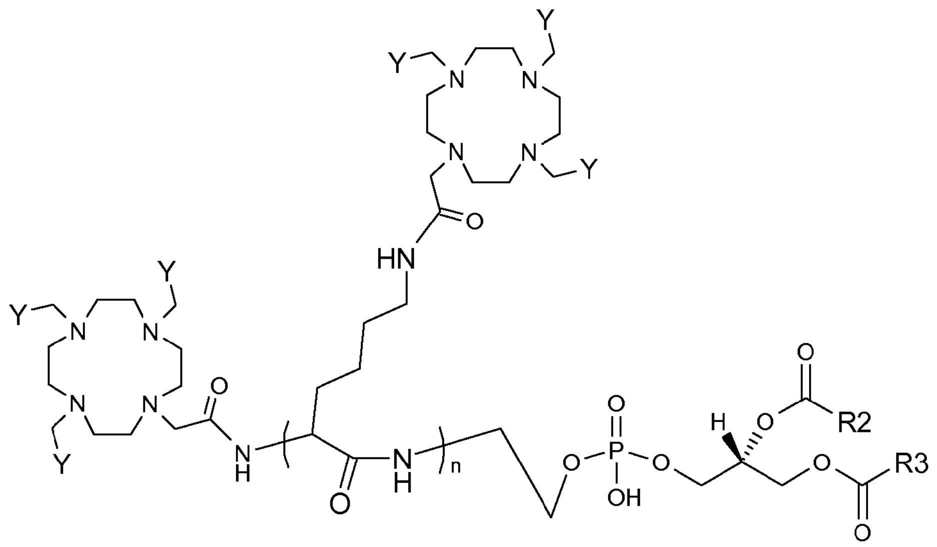

(VII)

In certain embodiments, the chelating agent derivatives of the present invention may comprise a chiral phospholipid group and therefore themselves be chiral. The chirality of the phospholipid group may be as shown in any of the structural formulae (III) to (VII). In some embodiments, the chelating agent derivatives of the present invention may further include chelated with the chelating agent, a metal such as e.g. a paramagnetic metal ion or a radionuclide metal. Illustrative paramagnetic metals include but are not limited to a lanthanide element of atomic numbers 58-70 or a transition metal of atomic numbers 21-29, 42 or 44, i.e., for example, scandium, titanium, vanadium, chromium, manganese, iron, cobalt, nickel, copper, molybdenum, ruthenium, cerium, praseodymium, neodymium, promethium, samarium, europium, gadolinium, terbium, dysprosium, holmium, erbium, thulium, and ytterbium, particularly Gd(III), Mn(II), iron, europium and/or dysprosium. Suitable radionuclides include the radioactive forms of, for example, Sm, Ho, Y, Pm, Gd, La, Lu, Yb, Sc, Pr, Tc, Re, Ru, Rh, Pd, Pt, Cu, Au, Ga, In, Sn, and Pb. The present invention also encompasses compositions comprising other exemplary radionuclides and paramagnetic ions known in the art.

The present invention also provides an imaging system comprising any chelating agent derivative as described hereinabove, together with equipment for performing imaging of an object that is adapted or suitable to detect the presence and/or the amount of said chelating agent derivative in said object. The imaging equipment can be selected from the group comprising or consisting of magnetic resonance imaging equipment, radionuclide imaging equipment, optical imaging equipment, X-ray imaging equipment, or a combination thereof.

In a second aspect, the present invention relates to a composition comprising chelating agent derivatives and lipophilic nanoparticles or microparticles.

As used herein and unless stated otherwise, the term lipophilic nanoparticles or microparticles relates to particulate carriers that are, at least on their surface, lipophilic and which can be suspended in a hydrophilic or aqueous medium. In some embodiments, they may have average diameters in the range of about 10 nm-100 μm, or about 50 nm-50 μm. However, for in vivo use, particles having diameters in the range of 50-500 nm, or 50-300 nm may be preferred. The lipophilic particles may be of a variety of compositions, including liposomes, which may be of various sizes and may be unilamellar or multilamellar, micelles, oil droplets, lipoproteins, such as HDL (high density lipoproteins), LDL (low density lipoproteins), IDL (intermediate density lipoproteins), VLDL (very low density lipoproteins), chylomicrons, fluorocarbon nanoparticles, microbubbles or nanobubbles, or any of a wide variety of particles in the above mentioned size range that are lipophilic at least at their surface. Thus, the surface of these nanoparticles or microparticles may comprise, in some embodiments, lipids or surfactants, or both.In one embodiment of this invention, the chirality of the phospholipid moiety of the chelating agent derivative may be the same as the chirality of the natural chiral lipids constituting the liposomes in which they are integrated. This particular embodiment may have the advantage of permitting a better packing, and therefore a better integration, of the phospholipid moiety of the chelating agent derivative into liposomes, themselves composed of natural chiral lipids.

In another embodiment of this invention, the lipophilic nanoparticles or microparticles may comprise a targeting agent and/or or a biologically active agent.

Targeting agents may comprise antibodies or immunospecific fragments thereof, ligands for receptors present on the desired target or tissue, molecules designed specifically to target cellular components such as those designed based on cyclic RGD peptides designed to target integrins and the like. The lipophilic micro- or nanoparticles themselves

may include reactive groups that can be coupled to targeting agents.

A variety of methods known in the art may be used to associate the targeting ligand and/or the targeting agent to the nanoparticles. These synthetic strategies may include the use of spacer groups such as polyethylene glycol or peptides, for example.

For example, for covalently binding or coupling the targeting ligand and/or other organic moiety to the outer layer of the lipophilic micro- or nanoparticle, various types of bonds and linking agents may be employed. Typical methods for forming such coupling include the formation of amides with the use of carbodiamides, or the formation of sulfide linkages through the use of unsaturated components such as maleimide derivatives. Other known coupling agents include, for example, glutaraldehyde, propanedial or butanedial, 2-iminothiolane hydrochloride, bifunctional N-hydroxysuccinimide esters such as disuccinimidyl suberate, disuccinimidyl tartrate, bis[2-(succinimidooxycarbonyloxy)ethyl] sulfone, heterobifunctional reagents such as N-(5-azido-2-nitrobenzoyloxy)succinimide, succinimidyl 4-(N-maleimidomethyl)cyclohexane-1-carboxylate, and succinimidyl 4-(p-maleimido-phenyl)butyrate, homobifunctional reagents such as 1,5-difluoro-2,4-dinitrobenzene, 4,4'-difluoro-3,3'-dinitrodiphenylsulfone, 4,4'-diisothiocyano-2,2'-disulfonic acid stilbene, p-phenylenediisothiocyanate, carbonylbis(L-methionine p-nitrophenyl ester), 4,4'-dithiobisphenylazide, erythritolbiscarbonate and bifunctional imidoesters such as dimethyl adipimidate hydrochloride, dimethyl suberimidate, dimethyl 3,3'-dithiobispropionimidate hydrochloride and the like. Linkage can also be accomplished by acylation, sulfonation, reductive amination, and the like. The ligand itself may be included in the surfactant layer if its properties are suitable therefor. For example, if the ligand contains a highly lipophilic portion, it may itself be embedded into the lipid/surfactant layer. Further, if the ligand is capable of direct adsorption to the layer, this will also effect coupling. For example, nucleic acids, because of their negative charge, adsorb directly to cationic surfactants.

The targeting agent may bind directly to the lipophilic micro- or nanoparticle, i.e., the ligand or antibody may be associated with the nanoparticle itself, as described above. Alternatively, indirect binding such as that effected through biotin/avidin may also be employed. Typically, in biotin/avidin mediated targeting, the ligand or antibody is not coupled to the emulsion itself, but rather coupled, in a biotinylated form, to the targeted tissue.

Radionuclides may for instance be therapeutic or diagnostic radionuclides; diagnostic imaging using such nuclides is well known and by targeting radionuclides to undesired tissue a therapeutic benefit may be realized as well. Typical diagnostic radionuclides include $^{99}$Tc, $^{95}$Tc, $^{111}$In, $^{62}$Cu, $^{64}$Cu, $^{67}$Ga, and $^{68}$ Ga, and therapeutic nuclides include $^{186}$Re, $^{188}$Re, $^{153}$Sm, $^{166}$Ho, $^{177}$Lu, $^{149}$Pm, $^{90}$Y, $^{212}$Bi, $^{103}$Pd, $^{109}$Pd $^{159}$Gd, $^{140}$La, $^{198}$Au, $^{199}$Au, $^{169}$Yb, $^{175}$Yb, $^{165}$Dy $^{166}$Dy, $^{67}$Cu, $^{105}$Rh, $^{111}$Ag, and $^{192}$Ir among others. The nuclide can be provided to a preformed emulsion in a variety of ways. For example, $^{99}$Tc-pertechnate may be mixed with an excess of stannous chloride and incorporated into the preformed emulsion of nanoparticles. Stannous oxinate can be substituted for stannous chloride. In addition, commercially available kits, such as the HM-PAO (exametazine) kit marketed as Ceretekg by Nycomed Amersham can be used. Means to attach various radioligands to the nanoparticles of the invention are understood in the art. As stated above, the radionuclide may not be an ancillary material, but may instead occupy the chelating agent in lieu of the paramagnetic ion when the composition is to be used solely for diagnostic or therapeutic purposes based on the radionuclide.

Other ancillary agents include known fluorophores such as but not limited to fluorescein, dansyl, and the like.

Included in the lipophilic carrier vehicle as ancillary agents, in some embodiments of the invention, may also be biologically active agents. These biologically active agents can be of a wide variety, including proteins, nucleic acids, pharmaceutical small organic molecules from various therapeutic classes, and the like. Thus, included among suitable pharmaceuticals are known antineoplastic agents, hormones, analgesics, anesthetics, neuromuscular blockers, antimicrobials or antiparasitic agents, antiviral agents, interferons, antidiabetics, antihistamines, antitussives, anticoagulants, and the like.

In all of the foregoing cases, whether the associated moiety is a targeting agent for a tissue or organ or is an ancillary agent, this moiety may be non-covalently associated with the lipophilic micro- or nanoparticle, may be directly coupled to the components of the micro- or nanoparticle, or may be coupled to said components through spacer moieties.

A multiplicity of micro- or nanoparticle may be used in the compositions of the invention, for example, liposomal particles. The literature describing various types of liposomes is vast and well known to the person skilled in the art. As the liposomes themselves are comprised of lipid moieties, the above-described lipids and surfactants are applicable in the description of moieties contained in the liposomes themselves. These lipophilic components can be used to couple to the chelating agent in a manner similar to that described above with respect to the coating on the nanoparticles having an inert core. Micelles are composed of similar materials, and this approach to coupling desired materials, and in particular, the chelating agents applies to them as well. Solid forms of lipids may also be used.

Preferably, the chelating agent derivative is at least partially embedded into the surface of the lipophilic nanoparticles or microparticles. In Fig. 1, a schematic representation of a composition according to an embodiment of the present invention is shown. The composition comprises a lipophilic particle having a membrane (1) in which chelating agent derivatives according to an embodiment of the present invention and a targeting agent is inserted.

In some embodiments of the present invention, the micro- or nanoparticle may contain at least 2,000 copies,

typically at least 5,000 copies, more typically at least 10,000 copies or at least 100,000 copies or at least 500,000 copies of the chelating agent derivative. For instance, in the case of liposomes, the number of metal complexes per liposome is a function of the nanoparticle size and the mole % of metal-based phospholipid in the liposome bilayer. For instance, the theoretical number of lipids and the theoretical number of Gd(III) complexes per liposome particle were calculated for sizes in the range of 5-500 nm and are shown in table 1 below.

Table 1

| Particle size (nm) | Lipids/liposome | Gd(III)/liposome[1] |
|---|---|---|
| 5.0 | 3.70E+02 | 9.24E+01 |
| 10.0 | 1.48E+03 | 3.70E+02 |
| 20.0 | 5.91E+03 | 1.48E+03 |
| 30.0 | 1.33E+04 | 3.33E+03 |
| 40.0 | 2.37E+04 | 5.91E+03 |
| 50.0 | 3.70E+04 | 9.24E+03 |
| 60.0 | 5.32E+04 | 1.33E+04 |
| 70.0 | 7.24E+04 | 1.81E+04 |
| 80.0 | 9.46E+04 | 2.37E+04 |
| 90.0 | 1.20E+05 | 2.99E+04 |
| 100.0 | 1.48E+05 | 3.70E+04 |
| 150.0 | 3.33E+05 | 8.32E+04 |
| 200.0 | 5.91E+05 | 1.48E+05 |
| 250.0 | 9.24E+05 | 2.31E+05 |
| 300.0 | 1.33E+06 | 3.33E+05 |
| 400.0 | 2.37E+06 | 5.91E+05 |
| 500.0 | 3.70E+06 | 9.24E+05 |
| [1] For 25 mole % of Gd(III)DOTA-based lipid. | | |

The present invention also includes an imaging system comprising a composition comprising any of the chelating agent derivatives described hereinabove and lipophilic nanoparticles or microparticles according to the present invention as described above, and equipment for performing imaging of an object such as an animal or human body, and adapted to detect the composition. The imaging equipment can be selected from the group comprising or consisting of magnetic resonance imaging equipment, radionuclide imaging equipment, optical imaging equipment, X-ray imaging equipment, or a combination thereof.

In a third aspect, the present invention relates to an imaging method comprising providing a chelating agent derivative to a location to be imaged. The location may be in-vivo or ex-vivo. The present invention also provides methods of magnetic resonance imaging using any of the chelating agent derivatives and compositions of the present invention, or mixtures thereof. In one embodiment, the present invention provides a method for imaging a sample, the sample including a chelating agent derivative as defined by any one of the above structural formulae, and imaging said sample. The imaging may be ex-vivo or in-vivo. The present invention also provides a method for imaging a sample including a composition comprising a chelating agent derivative represented by any one of the above structural formulae, and a lipophilic nanoparticle or a microparticle optionally comprising a targeting agent and/or a biologically active agent, and imaging said sample. In one embodiment, the sample is a tissue sample.

Examples of suitable imaging methods comprise, but are not limited to, magnetic resonance imaging, radionuclide imaging, optical imaging, X-ray imaging, or a combination thereof.

In a fourth aspect, the present invention relates to a method of manufacturing a chelating agent derivative. In an embodiment of this fourth aspect, the method comprises a step of reacting a phosphoethanolamine lipid or a derivative thereof with a chelating agent having at least one carboxylic acid group. If the chelating agent has more than one carboxylic acid group, the carboxylic groups in excess of one are preferably protected, using protection methods known in the art, in advance of said reaction step. In one embodiment of the present invention, at least one carboxylic acid

group of said chelating agent is coupled through a linker to said phosphoethanolamine lipid moiety, the method comprising the step of reacting said phosphoethanolamine lipid with said linker to first form a phosphoethanolamine lipid derivative, and then reacting said phosphoethanolamine lipid derivative with said chelating agent having at least one carboxylic acid group. Alternatively, the at least one carboxylic acid group of said chelating agent is coupled through a linker to said phosphoethanolamine lipid moiety, the method comprising the step of reacting said chelating agent with said linker to first form a chelating agent intermediate, and then reacting said chelating agent intermediate with said phosphoethanolamine lipid. In another embodiment, the method leads to the synthesis of a chelating agent derivative comprising more than one chelating moiety. This embodiment may comprise the step of reacting a phosphoethanolamine lipid with a linker selected from the group consisting of:

(a) oligopeptides or polypeptides comprising at least m-1 pending groups, m-1 of said pending groups being reactive toward chelating agents having at least one carboxylic acid group, and
(b) NHR"-$L_2$CONR'H, wherein $L_2$ is a hydrocarbon chain having from 2 to 40 carbon atoms, said hydrocarbon chain comprising at least m-1 pending groups, m-1 of said pending groups being each reactive toward chelating agents having at least one carboxylic acid group, said hydrocarbon chain optionally further comprising one or more oxo groups and/or heteroatoms independently selected from oxygen and nitrogen in the main chain,

wherein R' and R" are independently hydrogen or a $C_1$-$C_4$ alkyl chain. The product of this reaction may then be reacted with a suitable chelating agent having at least one carboxylic acid group.

An alternative embodiment of the manufacturing method consists in first reacting a chelating agent having at least one carboxylic acid group with a linker selected from the group consisting of:

(a) oligopeptides or polypeptides comprising at least m-1 pending groups, m-1 of said pending groups being reactive toward chelating agents having at least one carboxylic acid group, and
(b) NHR"$L_2$CONR'H, wherein $L_2$ is a hydrocarbon chain having from 2 to 40 carbon atoms, said hydrocarbon chain comprising at least m-1 pending groups, m-1 of said pending groups being each reactive toward chelating agents having at least one carboxylic acid group, said hydrocarbon chain optionally further comprising one or more oxo groups and/or heteroatoms independently selected from oxygen and nitrogen in the main chain,

wherein R' and R" are independently hydrogen or a $C_1$-$C_4$ alkyl chain. The product of this reaction may then be reacted with a phosphoethanolamine lipid or a derivative thereof having at least one functional group reactive toward said product. An optional step of any of the embodiments of the fourth aspect of the present invention consists in de-protecting, using deprotection methods well known in the art, the carboxylic acid groups that have optionally been originally protected.

Examples:

Unless stated otherwise, all reagents and chemicals were obtained from commercial sources and used without further purification. The following abbreviations are used: DCM = dichloromethane, DMF = dimethylformamide, DiPEA = diisopropyl ethylamine, MeOH = methanol, TFA = trifluoroacetic acid, HBTU = O-(Benzotriazol-1-yl)-*N,N,N',N'*-tetramethyl-uronium hexafluoro-phosphate. 1,2-Dipalmitoyl-*sn*-glycero-3-phosphoethanolamine **(4a)** and 1,2-distearoyl-*sn*-glycero-3-phosphoethanolamine **(4b)** were purchased from Alexis Biochemicals. All hygroscopic compounds were stored in a desiccator over $P_2O_5$. DiPEA was stored over KOH pellets.

$^1$H-NMR, $^{13}$C-NMR and $^{31}$P-NMR spectra were respectively recorded on a Varian Gemini-2000 300 MHz spectrometer, a Varian Mercury Vx 400 MHz spectrometer, and a Varian Unity Inova 500 MHz spectrometer at 298 K. Chemical shifts are given in the form of ppm (δ) values. Infrared spectra were recorded on a Perkin-Elmer 1605 FT-IR spectrometer. MALDI-TOF spectra were obtained on a Perspective Biosystems Voyager DE-Pro. The HPLC system was coupled to an evaporative light scattering detector (ELSD). All HPLC runs were executed with a 0.5 mL/min flow rate using a Kromasil C18 column (150 × 3.2 mm).

Example 1: Synthesis of Gd(III)DOTA-$C_6$-phospholipids **21a** ($R_2$=$R_3$$C_{15}H_{31}$)

A chelating agent derivative **21a** represented by the structural formula (VIII) wherein $R_2$ and $R_3$ are palmitoyl groups ($C_{15}H_{31}$) has been synthesized as described in Fig. 2.

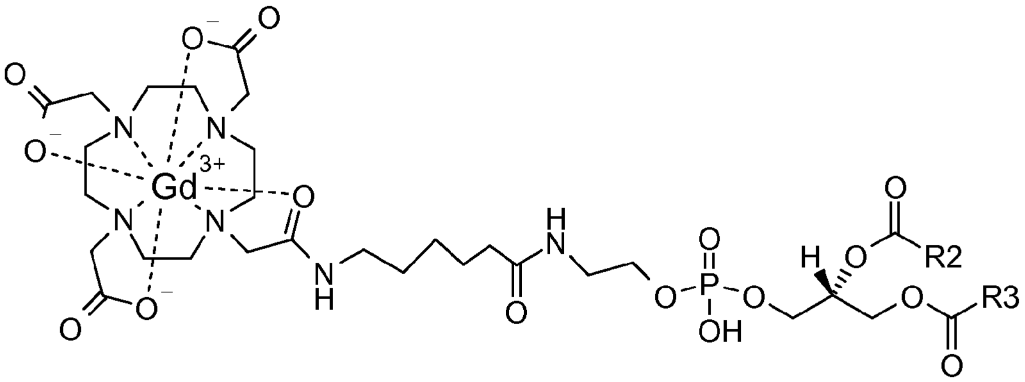

(VIII)

In a first step, the chelating moiety **15** derived from DOTA was synthesised according to Heppeler, A. et al., M. Chemistry-A European Journal (1999) 5, 1974-1981. In step (a) of Fig. 2, the DOTA-based building block **15** (0.171 g, 0.30 mmol) was added to a solution of HBTU (0.113 g, 0.30 mmol) and DiPEA (174 μL, 1.00 mmol) in dry DMF (1.0 mL). The mixture was stirred for 5 minutes at room temperature under an atmosphere of argon. The obtained yellowish solution was added to a solution of the phospholipid **8a** (0.200 g, 0.249 mmol) in dry 1:2 v/v DMF/$CHCl_3$ (9 mL) under an atmosphere of argon. Subsequently, the solution was heated to 40 °C and stirring was continued overnight. The solution was concentrated under reduced pressure, dissolved in $CHCl_3$, and washed with citric acid buffer pH=4 (2 × 10 mL). The organic phase was washed with 1:10 v/v 0.1 N NaOH (aq)/saturated NaCl (aq) (2 × 10 mL) and dried over anhydrous $Na_2SO_4$. After filtration, the organic phase was concentrated in vacuo. The crude product was further purified using column chromatography ($SiO_2$, $CHCl_3$ → 2:5 v/v EtOH/$CHCl_3$) to give the chelating agent derivative **19a** (0.155 g, 0.114 mmol) in 46% yield which was characterized as follows:

- $^1$H-NMR ($CDCl_3$): δ = 9.3 (br t, 1H, NH), 8.5 (br t, 1H, NH), 5.2 (m, 1H, H2), 4.4-4.3 (m, 1H, $J_1$ = 12.0 Hz, $J_2$ = 3.3 Hz, H1), 4.2-4.15 (m, 1H, $J_1$ = 11.8 Hz, $J_2$ = 6.3 Hz, H1'), 4.1-3.9 (m, 4H, H3 {2H} + $POCH_2CH_2NHCO$ {2H}), 3.6-2.0 (br m, 34 H, $NCH_2CH_2N$ {16H} + $NCH_2CO$ {8H} + $POCH_2CH_2NHCO$ {2H} + $NCH_2CONHCH_2CH_2$ {2H} + $CH_3(CH_2)_{13}CH_2CO$ {4H} + $CH_2CH_2CONHCH_2$ {2H}), 1.7-1.0 (m, 85H), 0.88 (t, 6H, J = 6.7 Hz, $CH_2CH_3$);
- FT-IR (ATR): ν (cm$^{-1}$) 3249, 2923, 2853, 1731, 1664, 1556, 1456, 1368, 1225, 1162, 1106, 1067 and 808.
- MALDI-TOF (negative mode): m/z $[C_{71}H_{135}N_6O_{16}P_1 - H]^-$ Calculated. 1358.0 Da.; Observed. 1357.9 Da.

In step (b) of Fig. 2, TFA (2 mL) was added under an atmosphere of argon to a solution of **19a** (0.115 g, 0.085 mmol) in freshly distilled DCM (4 mL). The solution was vigorously stirred for 1 hour at room temperature. The solution was concentrated under reduced pressure (below 30°C), dissolved in 1:2 v/v TFA/DCM (6 mL) and stirring was continued overnight at room temperature. Subsequently, the solution was concentrated under reduced pressure, co-evaporated with toluene (3 × 10 mL), $CHCl_3$ (3 × 10 mL) to give **20a** (in quantitative yield) as a yellowish solid. The $^1$H-NMR spectrum in $CDCl_3$ showed a set of broad signals. Moreover, the $^1$H-NMR spectrum confirmed the successful removal of the tert-butyl ester groups as indicated by the absence of signals at 1.5 ppm.

- FT-IR (ATR): ν (cm$^{-1}$) 3334, 2923, 2853, 1720, 1673, 1465, 1202, 1139 and 801.
- MALDI-TOF (positive mode): m/z $[C_{59}H_{111}N_6O_{16}P_1 + H]^+$ Calculated. 1191.8 Da.; Observed. 1191.8 Da.; $[M + Na]^+$ Calcd. 1213.8 Da.; Obsd. 1213.8 Da.; $[M + 2Na]^+$ Calcd. 1236.8 Da.; Obsd. 1236.8 Da.

In step (c) of Fig. 2, to a solution of **20a** (107 mg, 89.9 μmol) in $CHCl_3$ (6.48 mL) was added a solution of Gd (III)$(OAc)_3$·5.1 $H_2O$ (34.5 mg, 80.9 μmol, 0.9 equivalents) in 10:1 v/v MeOH/$H_2O$ (3.17 mL). Subsequently, two droplets of pyridine were added and the solution was stirred overnight at room temperature. The formation of the Gd(III) complex was monitored employing analytical RP HPLC. Since the reaction was performed with a small excess of DOTA-phospholipid, the HPLC elution profile showed a large single peak for the Gd(III) complex and a small single peak for the DOTA-$C_6$-phospholipid. The solution was concentrated under reduced pressure and co-evaporated with 1:1 v/v MeOH/toluene (2 × 10 mL) and $CHCl_3$ (2 × 10 mL) to yield Gd(III) complex **21a** (119 mg) as a yellowish solid which was characterized as follows:

- FT-IR (ATR): ν (cm$^{-1}$) 3318 (-NH stretch), 2924, 2854, 1678, 1602, 1444, 1413, 1199, 1136, 1084 and 780.
- ESI-MS (direct injection, negative mode): m/z $[C_{59}H_{107}N_6O_{16}P_1Gd_1 - H]^-$ Calculated. 1344.7 Da.; Observed. 1345.0

Da.

Example 2: Synthesis of Gd(III)DOTA-$C_6$-phospholipids **21b** (R2=R3=$C_{17}H_{35}$).

A chelating agent derivative **21b** represented by the structural formula (VIII) wherein $R_2$ and $R_3$ are stearyl groups ($C_{17}H_{35}$) has been synthesized as described in Fig. 2. This compound was prepared using the same procedure as described for **19a,** but starting with **8b.**

Example 3: Synthesis of Gd(III)DOTA-phospholipids **18a** (R2=R3=$C_{15}H_{31}$).

A chelating agent derivative **18a** represented by the structural formula (IX) wherein $R_2$ and $R_3$ are palmitoyl groups ($C_{15}H_{31}$) has been synthesized as described in Fig. 3.

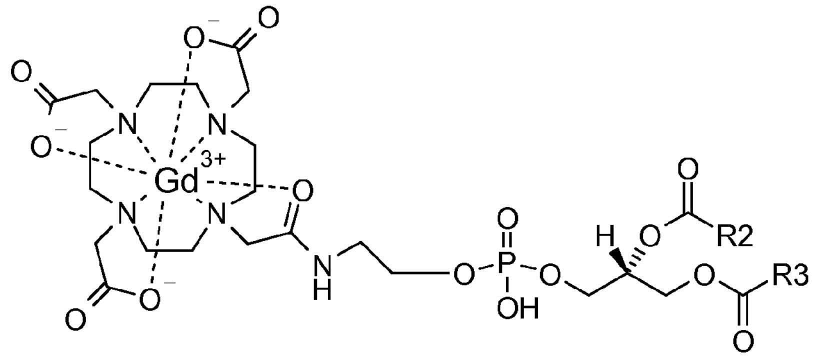

(IX)

In step (a) of Fig. 3, DOTA-based building block **15** (0.166 g, 0.289 mmol) was added to a solution of HBTU (0.109 g, 0.287 mmol) and DiPEA (202 μL, 1.16 mmol) in dry DMF (1.0 mL). The mixture was stirred for 5 minutes at room temperature under an atmosphere of argon. The obtained yellowish solution was added to a suspension of phospholipid **4a** (0.200 g, 0.289 mmol) in dry 1:2 v/v DMF/$CHCl_3$ (3 mL) under an atmosphere of argon. The temperature was increased to 50 °C for 10 minutes. The obtained solution was stirred for 2 hours at 50 °C. After cooling down to 40 °C, the reaction was continued overnight. The clear solution was concentrated under reduced pressure, dissolved in $CHCl_3$ (70 mL), and washed with 0.1 N NaOH (aq). The organic phase was dried over anhydrous $Na_2SO_4$. After filtration, the organic phase was evaporated to dryness. The remaining solid was purified using column chromatography ($SiO_2$, gradient DCM→2:1 v/v DCM/EtOH) to give **16a** (0.120 g, 0.096 mmol) in 33% yield which was characterized as follows:

- $^1$H-NMR ($CDCl_3$):δ = 9.44 (br t, 1H, N<u>H</u>), 5.24 (m, 1H, H2), 4.46-4.42 (m, 1H, $J_1$= 12 Hz, $J_2$= 3.4 Hz, H1), 4.24-4.20 (m, 1H, $J_1$= 12 Hz, $J_2$ = 6.3 Hz, H1'), 4.1-3.9 (m, 4H, H3{2H} + POC<u>H</u>$_2$CH$_2$NHCO{2H}), 3.6-2.0 (br m, 26H, NC<u>H</u>$_2$C<u>H</u>$_2$N {16H} + NC<u>H</u>$_2$CO {8H} + POCH$_2$C<u>H</u>$_2$NHCO {2H}), 2.31-2.20 (m, 4H, $CH_3(CH_2)_{13}$C<u>H</u>$_2$CO), 1.86-1.00 (m, 79H), 0.88 (t, 6H, J=7.0 Hz, CH$_2$C<u>H</u>$_3$). The assignment of the $^1$H-NMR spectrum is confirmed by $^1$H,$^1$H-COSY.
- $^{13}$C-NMR ($CDCl_3$, 125 MHz): δ = 173.5 (C=O), 173.1 (C=O), 172.3 (C=O), 171.1 (C=O), 81.9 (<u>C</u>(CH$_3$)$_3$, 70.8, 70.7, 63.3-62.9 (multiple signals), 56.3, 55.7, 54-47 (set oftwo broad signals), 41.6, 34.3, 34.2, 31.9, 29.7-29.0 (multiple signals), 27.9, 24.9, 22.7 and 14.1.
- FT-IR (ATR): ν (cm$^{-1}$) 3261 (-NH stretch), 2924, 2853, 1732, 1667, 1563, 1456, 1380, 1368, 1312, 1224, 1164, 1105 and 1066.
- MALDI-TOF (positive mode): m/z [$C_{65}H_{124}N_5O_{15}P_1$ + H]$^+$ Calculated. 1246.9 Da.; Observed. 1247.0 Da.; [M + Na]$^+$ Calculated. 1268.9 Da.; Observed. 1268.9 Da. MALDI-TOF (negative mode): m/z [M - H]$^-$ Calculated. 1244.9 Da.; Observed. 1244.8 Da.

In step (b) of Fig. 3, to a solution of tert-butyl ester protected DOTA-based phospholipid **16a** (0.120 g, 0.096 mmol) in DCM (4 mL) was added TFA (2 mL) under an atmosphere of argon. The solution was vigorously stirred for 2 hours at room temperature. The solution was concentrated under reduced pressure (below 30 °C), dissolved in 1:2 v/v TFA/DCM (6 mL), and stirring was continued overnight at room temperature. Subsequently, the solution was concentrated under reduced pressure, co-evaporated with toluene (3 × 10 mL), and $CHCl_3$ (2 × 10 mL) to yield **17a** (in quantitative yield) as a yellowish solid. The $^1$H-NMR spectrum of **17a** in $CDCl_3$ exhibits broadening of all signals. Moreover, the $^1$H-NMR spectrum clearly indicated the successful removal of the tert-butyl ester groups by the absence of signals at 1.46

ppm.

- $^{1}H$-NMR ($CDCl_3$): δ = 5.18 (br. m, 1H, H2), 4.4-2.4 (br. m, 32H), 2.27 (m, 4H, $CH_3(CH_2)_{13}C\underline{H_2}CO$), 1.25-1.15 (m, 52H), 0.88 (t, 6H, J = 6.8 Hz, $CH_2C\underline{H_3}$).
- FT-IR (ATR): ν ($cm^{-1}$) 3388 (-NH stretch), 3096, 2918, 2851, 1733, 1677, 1467, 1382, 1202, 1134 and 1055.
- MALDI-TOF (positive mode): m/z $[C_{53}H_{100}N_5O_{15}P_1 + H]^+$ Calcd. 1078.7 Da.; Obsd. 1078.7 Da.; $[M + Na]^+$ Calcd. 1100.7 Da.; Obsd. 1100.7 Da.; $[M + 2Na]^+$ Calcd. 1122.7 Da.; Obsd. 1122.7 Da.
- MALDI-TOF (negative mode): m/z $[M - H]^-$ Calcd. 1076.7 Da.; Obsd. 1076.6 Da.

In step (C) of Fig. 3, To a solution of **17a** (94 mg, 87.3 μmol) in $CHCl_3$ (6.48 mL) was added a solution of Gd (III)$(OAc)_3 \cdot 5.1\ H_2O$ (33.5 mg, 78.6 μmol, 0.9 equivalents) in 10:1 v/v $MeOH/H_2O$ (3.17 mL). Subsequently, two droplets of pyridine were added and the solution was stirred overnight at room temperature. The formation of the Gd(III) complex was monitored employing analytical RP HPLC. Since the reaction was performed with a small excess of DOTA-phospholipid, the HPLC elution profile showed a large single peak for the Gd(III) complex and a small single peak for the DOTA-phospholipid. The solution was concentrated under reduced pressure and co-evaporated with 1:1 v/v MeOH/ toluene (2 × 10 mL) and $CHCl_3$ (2 × 10 mL) to yield Gd(III) complex **18a** (117 mg) as a yellowish solid which was characterized as follows:

- FT-IR (ATR): ν ($cm^{-1}$) 3386 (-NH stretch), 2922, 2853, 1733, 1683, 1606, 1403, 1320, 1201, 1085, 1003, 939, 800 and 719.
- ESI-MS (direct injection, negative mode): m/z $[C_{53}F[_{97}N_5O_{15}P_1Gd_1 - H]^-$ Calcd. 1231.6 Da.; Obsd. 1231.9 Da.

<u>Example 4: Synthesis of Gd(III)DOTA-phospholipids **18b** ($R_2$=$R_3$=$C_{17}H_{35}$).</u>

A chelating agent derivative **18b** represented by the structural formula (IX) wherein R2 and R3 are stearyl groups ($C_{17}H_{35}$) has been synthesized as described in Fig. 3.

In step (a) of Fig. 3, DOTA-based building block **15** (0.453 g, 0.791 mmol) was added to a solution of HBTU (0.300 g, 0.792 mmol) and DiPEA (550 μL, 3.14 mmol) in dry DMF (3.0 mL). The mixture was stirred for 5 minutes at room temperature under an atmosphere of argon. The obtained yellowish solution was added to a suspension of phospholipid **4b** (0.589 g, 0.787 mmol) in dry 1:2 v/v $DMF/CHCl_3$ (9 mL) under an atmosphere of argon. The temperature was increased to 50 °C and after 10 minutes a clear solution was obtained. This solution was stirred for another 2 hours at 50 °C. After cooling, the solution was stirred overnight at room temperature. The solution was concentrated under reduced pressure, dissolved in $CHCl_3$, and washed with citric acid buffer pH=4 (2 × 10 mL). The organic phase was washed with 1:10 v/v 0.1 N NaOH (aq)/saturated NaCl (aq) (2 × 10 mL) and dried over anhydrous $Na_2SO_4$. After filtration, the organic phase was concentrated in vacuo. The crude product was triturated with 1:1 v/v $MeOH/H_2O$ and stored at 4 °C. Centrifugation of the mixture at 2800 rpm for 5 minutes furnished **16b** (0.451 g, 0.346 mmol) as a yellowish wax in 44% yield which was characterized as follows.

- $^{1}H$-NMR ($CDCl_3$): δ = 9.6 (br t, 1H, N$\underline{H}$), 5.2 (m, 1H, H2), 4.5-4.4 (m, 1H, $J_1$ = 12.0 Hz, $J_2$ = 3.4 Hz, H1), 4.3-4.2 (m, 1H, $J_1$ = 12 Hz, $J_2$ = 6.4 Hz, H1'), 4.1-3.9 (m, 4H, H3 {2H} + $POC\underline{H_2}CH_2NHCO$ {2H}), 3.6-2.0 (br m, 26 H, $NC\underline{H_2}C\underline{H_2}N$ {16H} + $NC\underline{H_2}CO$ {8H} + $POCH_2CH2NHCO$ {2H}), 2.3-2.2 (m, 4H, $CH_3(CH_2)_{15}C\underline{H}2CO$),1.7-1.0 (m, 87H), 0.9 (t, 6H, J = 6.8 Hz, $CH_2C\underline{H}3$). The assignment of the $^{1}H$-NMR spectrum is confirmed by $^{1}H,^{1}H$-COSY.
- $^{13}C$-NMR ($CDCl_3$, 125 MHz): δ = 173.5 (C=O), 173.1 (C=O), 173.0 (C=O), 170.9 (C=O), 81.9 ($\underline{C}(CH_3)_3$, 70.8-70.7, 63.3-63.0 (multiple signals), 56.4, 55.7, 54-47 (set of two broad signals), 41.9, 34.4, 34.2, 31.9, 29.7-29.0 (multiple signals), 27.9, 24.9, 22.7, 14.1.
- $^{31}P$-NMR (CDCl3): δ = 1.4.
- FT-IR (ATR): ν ($cm^{-1}$) 3212 (-NH stretch), 2924, 2853, 1729, 1668, 1567, 1456, 1381, 1369, 1312, 1227, 1163, 1066 and 908.
- MALDI-TOF (positive mode): m/z $[C_{69}H_{132}N_5O_{15}P_1 + H]^+$ Calcd. 1302.95 Da.; Obsd. 1302.82 Da.; $[M+Na]^+$ Calcd. 1324.9 Da.; Obsd. 1324.8 Da.
- MALDI-TOF (negative mode): m/z $[M - H]^-$ Calcd. 1300.9 Da.; Obsd. 1300.7 Da.

In step (b) of Fig. 3, to a solution of **16b** (0.451 g, 0.346 mmol) in freshly distilled DCM (8 mL) was added TFA (4 mL) under an atmosphere of argon. The solution was vigorously stirred for 2 hours at room temperature. The solution was concentrated under reduced pressure (below 30 °C), dissolved in 1:2 v/v TFA/DCM (12 mL), and stirring was continued overnight at room temperature. Subsequently, the solution was concentrated under reduced pressure, co-evaporated with toluene (3 × 10 mL), $CHCl_3$ (2 × 10 mL) and precipitated in $CH_3CN$ to yield **17b** (0.309 g, 0.273 mmol) as a yellowish solid which was characterized as follows. The $^{1}H$-NMR spectrum in $CDCl_3$ exhibits broadening of all

signals. Additionally, $^1$H-NMR spectroscopy confirmed the successful removal of the tert-butylester groups indicated by the absence of signals at 1.46 ppm.

- $^1$H-NMR ($CDCl_3$): δ = 5.2 (br. m, 1H, H2), 4.4-2.4 (br. m, 32H), 2.3 (m, 4H, $CH_3(CH_2)_{15}C\underline{H_2}CO$), 1.3-1.1 (m, 60H), 0.9 (t, 6H, J = 6.4 Hz, $CH_2C\underline{H_3}$).
- $^{31}$P-NMR (CDCl3): δ = -1.8.
- FT-IR (ATR): ν ($cm^{-1}$) 3356, 2918, 2851, 1728, 1675, 1467, 1387, 1163 and 1049.
- MALDI-TOF (positive mode): m/z $[C_{57}H_{108}N_5O_{15}P_1 + H]^+$ Calcd. 1134.8 Da.; Obsd. 1134.8 Da.; $[M + Na]^+$ Calcd. 1156.8 Da.; Obsd. 1156.8 Da.; $[M + 2Na]^+$ Calcd. 1178.7 Da.; Obsd. 1178.8 Da.
- MALDI-TOF (negative mode): m/z $[M - H]^-$ Calcd. 1132.5 Da.; Obsd. 1132.6 Da.

The compound **18b** was prepared using the same procedure (step (c) of Fig. 3) as described for **18a,** but starting with **17b,** and was characterized as follows:

- FT-IR (ATR): ν ($cm^{-1}$) 3253, 2922, 2852, 1735, 1595, 1465, 1405, 1319, 1201, 1085 and 1064.
- ESI-MS (negative mode): m/z $[C_{57}H_{105}N_5O_{15}P_1Gd_1 - H]^+$ Calcd. 1287.7 Da.; Obsd. 1287.7 Da.

Example 5: Preparation of liposomal MRI contrast agents

Liposomes with an average diameter of 100-200 nm were formed by the lipid film hydration technique coupled with sequential extrusion as described in the literature [Strijkers, G. J. et al. Magnetic Resonance Materials in Physics Biology and Medicine 2005, 18, 186-192]. The composition of the liposomes is depicted in Table 2 First, the phospholipids and cholesterol was dissolved in $CHCl_3$ and MeOH. The solvent was gently removed under reduced pressure. The film was hydrated using HEPES buffered saline (HBS) of pH 7.4 under heating at 60°C and some shaking for approximately 30 minutes. The resulting milky dispersion was extruded several times through PC membrane filters with pore diameters of 200 and subsequently 100 nm, using a 10 ml LIPEX thermobarrel extruder (Northern Lipids Inc., Vancouver Canada) at 60°C.

Table 2 – composition of the liposome samples in mole-%.

| Exper iment | Cholesterol | DSPC | DPPC | PEG2000 -DSPE | Gd(III)DOTA -C6-DPPE (**21a**) | Gd(III)DOTA -C6-DSPE (**21b**) | Gd(III)DOTA -DPPE (**18a**) | Gd(III)DOTA -DSPE (**18b**) |
|---|---|---|---|---|---|---|---|---|
| 1 | 33 | | 37 | 5 | 25 | | | |
| 2 | 33 | 37 | | 5 | | 25 | | |
| 3 | 33 | | 37 | 5 | | | 25 | |
| 4 | 33 | 37 | | | | | | 25 |

Wherein DSPC stands for distearoylphosphatidylcholine, DPPC stands for dipalmitoylphosphatifylcholine and PEG2000-DSPE stands for polyethylene glycoldistearoylphosphatidylethanolamine.

Example 6: Characterization of the prepared liposomal MRI contrast agents

The mean diameter of the liposomes was determined with dynamic light scattering. The shape and the size distribution of the particles was studied in detail with cryotransmission electron microscopy (cryoTEM). For this purpose, thin films of the samples were vitrified using a Vitrobot (FEI Company, Acht; co-developed with Maastricht University).The concentration of Gd(III) was determined by means of Inductively Coupled Plasma-Atomic Emission Spectrometry (ICP-AES).

Example 7: Relaxivities of the prepared liposomal MRI contrast agents

The relaxivities $r_1$ and $r_2$ of an MRI contrast agent give a measure for the enhancement of the longitudinal and transversal relaxation rate of the bulk water protons, respectively.

$$\frac{1}{T_{1,2}} = \frac{1}{T_{1,2\text{dia}}} + r_{1,2}[\text{CA}]$$

Where $T_{1,2}$ and $T_{1,2}$ dia are the longitudinal and transvere relaxation times of water in the presence and in the absence of the contrast agent, respectively.[3;4] The relaxivities for the Gd(III)DOTA-containing liposomes were determined by measuring $T_1$ and $T_2$ as a function of concentration (T=293 K, 60 MHz). $T_1$ was measured using an inversion recovery sequence with variable inversion times, whereas $T_2$ was obtained using a spin-echo sequence with varying echo times. The relaxivities were determined from linear fits of $1/T_1$ and $1/T_2$ as a function of Gd(III) concentration. The results of the measurements are listed in Table 3.3. Gd(III)DOTA-phospholipid containing liposomes (experiments 1-4) display high values for the longitudinal and transverse relaxivities per gadolinium. These values are significantly higher compared to the relaxivities of the native Gd(III)DOTA complex ($r_1$ ~ 4 $mM^{-1}$ $S^{-1}$).

Table 3. Longitudinal and transverse relaxivities of liposomes with Gd(III)DOTA-based lipids incorporated in the bilayer

| Experiment | Gd(III) complex | $r_1$ ($mM^{-1}s^{-1}$) | $r_2$ ($mM^{-1}s^{-1}$) | $r_2/r_1$ |
|---|---|---|---|---|
| 1 | **21a** | 18.6 | 29.5 | 1.59 |
| 2 | **21b** | 23.1 | 37.6 | 1.63 |
| 3 | **18a** | 15.4 | 23.0 | 1.49 |
| 4 | **18b** | 16.1 | 26.9 | 1.67 |

It is to be understood that although preferred embodiments, specific constructions and configurations, as well as materials, have been discussed herein for devices according to the present invention, various changes or modifications in form and detail may be made without departing from the scope and spirit of this invention.

## Claims

**1.** A chelating agent derivative represented by the structural formula :

$$(Ch\text{-}CO)_m\text{-}NR''\text{-}L_1\text{-}CH_2\text{-}CH_2\text{-}O\text{-}P(=O)(X)OCH_2CH(OCOR_2)(CH_2OCOR_3) \qquad (I)$$

wherein

- $R_2$ and $R_3$ are independently selected from the group consisting of $C_{12}$-$C_{22}$ alkyl and $C_{12}$-$C_{22}$ alkenyl,
- Ch is a chelating moiety derived from ethylenediamine tetraacetic acid (EDTA), 1,4,10,13-tetraoxa-7,16-diazacyclooctadecane-7 (ODDA) 16-diacetate,*N*-2-(azol-1-yl)ethyliminodiacetic acid, 1,4,7,10-tetraaza-cyclododecane-N,N',N'',N'''-tetraacetic acid (DOTA), 1,7,13-triaza-4,10,16-trioxacyclooctadecane-N,N',N''-triacetate (TTTA), tetraethyleneglycol-1,5,9-triazacyclododecane-N,N',N'',-tris(methylenephosphonic acid) (DOTRP) or 1,4,7,10-tetraazacyclododecane-α,α',α'',α'''-tetramethyl-N,N',N'',N'''-tetraacetic acid (DOTMA),

- m is an integer ranging from 1 to 10,
wherein

- when m=1, $L_1$ is a linker selected from the group consisting of a single bond, oligopeptides, polypeptides, oligoethylene glycol, polyethylene glycol or $L_2$CONR' wherein $L_2$ is a hydrocarbon chain having from 2 to 40 carbon atoms, said hydrocarbon chain optionally comprising one or more oxygen atoms in the main chain and/or being further optionally substituted with one or more substituents independently selected from the group consisting of $C_{1-4}$ alkyl and $C_{1-4}$ alkoxy andoptionally comprising at least one primary amino group,
- when m is 2 to 10, $L_1$ is a linker selected from the group consisting of
- (a) oligopeptides or polypeptides comprising at least m-1 pending groups, m-1 of said pending groups being each directly coupled to a Ch-CO- group, and
- (b) $L_2$CONR', wherein $L_2$ is a hydrocarbon chain having from 2 to 40 carbon atoms, said hydrocarbon chain comprising at least m-1 pending groups, m-1 of said pending groups being each directly coupled to a Ch-CO- group, said hydrocarbon chains optionally further comprising one or more oxo groups and/or heteroatoms independently selected from oxygen and nitrogen in the main chain,
- R' and R" are independently H or a $C_1$-$C_4$ alkyl chain,
- X is OH or O-$M^+$, and
- $M^+$ is a counter-ion,

a salt or a stereoisomeric form thereof.

2. The chelating agent derivative of claim 1, wherein m is 2 to 10 and $L_1$ is an oligopeptide or a polypeptide comprising at least m-1 lysine residues.

3. The chelating agent derivative of claim 2, wherein m-1 of said lysine residues are each directly coupled to a (Ch-CO)- group.

4. The chelating agent derivative of claim 3, wherein $L_1$ is an oligolysine or a polylysine comprising lysine residues, wherein m-1 of said lysine residues are each directly coupled to a (Ch-CO)- group.

5. The chelating agent derivative of claim 1, wherein m=1 and $L_2$ is an oligo- or a polyethyleneglycol chain.

6. The chelating agent derivative of any of claim 1 to 5, having the following general formula :

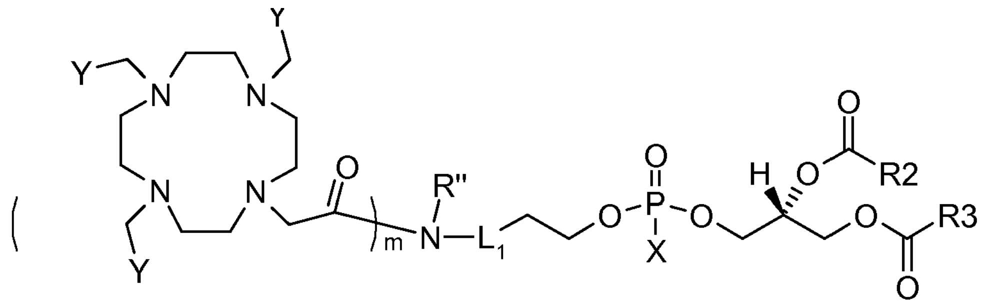

(III)

wherein:

- Y is selected from the group consisting of a carboxylic group, a carboxylate ion or salt, a methyl ester, methoxymethyl ester, methylthiomethyl ester, tetrahydropyranyl ester, benzyloxymethyl ester, phenacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester, 2-(9,10-dioxo)anthrylmethyl ester, piperonyl ester, trimethylsilyl ester, t-butyldimethylsilyl ester, S-t-butyl ester, and 2-alkyl-1,3-oxazolines,
- X is OH or $O^-M^+$,
- $M^+$ is a counter-ion,
- m is an integer ranging from 1 to 10, provided that $L_1$ is not a single bond when m is higher than 1,
- $L_1$ is a single bond or is a linker represented by the structural formula

$L_2$(CO)-NR'-, (II)

wherein

- when m=1, $L_1$ is a linker selected from the group consisting of a single bond, oligopeptides, polypeptides, oligoethylene glycol, polyethylene glycol or $L_2$CONR' wherein $L_2$ is a hydrocarbon chain having from 2 to 40 carbon atoms, said hydrocarbon chain optionally comprising one or more oxygen atoms in the main chain and/or being further optionally substituted with one or more substituents independently selected from the group consisting of $C_{1-4}$ alkyl and $C_{1-4}$ alkoxy and optionally comprising at least one primary amino group,
- when m is 2 to 10, $L_1$ is a linker selected from the group consisting of
- (a) oligopeptides or polypeptides comprising at least m-1 pending groups, m-1 of said pending groups being each directly coupled to a Ch-CO- group, and
- (b) $L_2$CONR', wherein $L_2$ is a hydrocarbon chain having from 2 to 40 carbon atoms, said hydrocarbon chain comprising at least m-1 pending groups, m-1 of said pending groups being each directly coupled to a Ch-CO- group, said hydrocarbon chain optionally further comprising one or more oxo groups and/or heteroatoms independently selected from oxygen and nitrogen in the main chain,
- R' and R" are independently H or a $C_1$-$C_4$ alkyl chain,
- X is OH or $O^-M^+$, and
- $M^+$ is a counter-ion, a salt or a stereoisomeric form thereof.

**7.** The chelating agent derivative of claim 1 represented by one of the following structural formulae:

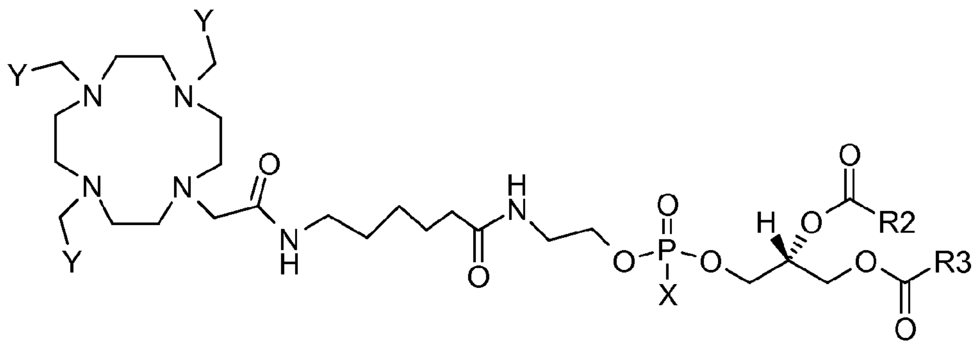

(X)

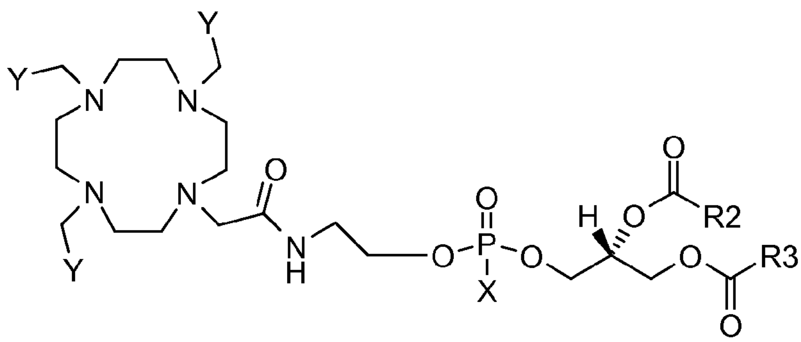

(XI)

wherein:

- X is OH or $O\text{-}M^+$,
- Y is selected from the group consisting of a carboxylic acid group, a carboxylate ion or salt, a methyl ester, methoxymethyl ester, methylthiomethyl ester, tetrahydropyranyl ester, benzyloxymethyl ester, phenacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester,

2-(9,10-dioxo)anthrylmethyl ester, piperonyl ester, trimethylsilyl ester, t-butyldimethylsilyl ester, S-t-butyl ester, and 2-alkyl-1,3-oxazolines, and
- $R_2$ and $R_3$ are independently selected from the group consisting of $C_{12}$-$C_{22}$ alkyl and $C_{12}$-$C_{22}$ alkenyl.

**8.** The chelating agent derivative of any of claims 1 to 6 represented by one of the following structural formula:

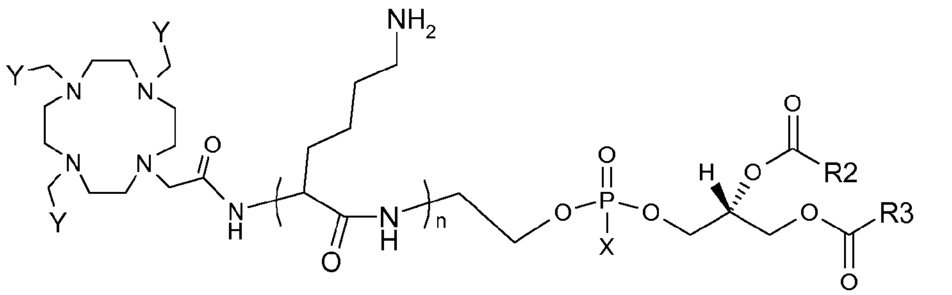

(VI)

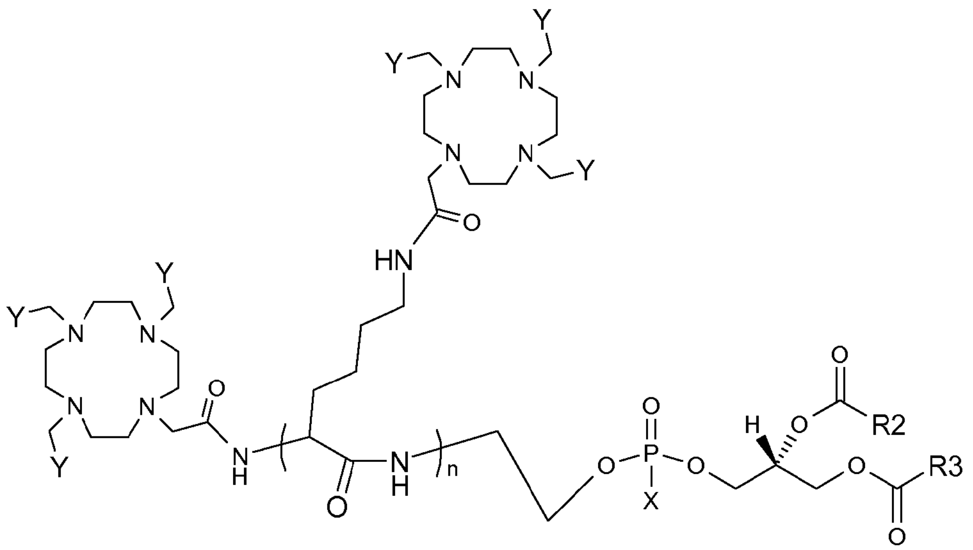

(VII)

wherein:

- X is OH or O-M+,
- Y is selected from the group consisting of a carboxylic group, a carboxylate ion or salt, a methyl ester, methoxymethyl ester, methylthiomethyl ester, tetrahydropyranyl ester, benzyloxymethyl ester, phenacyl ester, N-phthalimidomethyl ester, 2,2,2-trichloroethyl ester, 2-haloethyl ester, 2-(p-toluenesulfonyl)ethyl ester, t-butyl ester, cinnamyl ester, benzyl ester, triphenylmethyl ester, bis(o-nitrophenyl)methyl ester, 9-anthrylmethyl ester, 2-(9,10-dioxo)anthrylmethyl ester, piperonyl ester, trimethylsilyl ester, t-butyldimethylsilyl ester, S-t-butyl ester, and 2-alkyl-1,3-oxazolines, and
$R_2$ and $R_3$ are independently selected from the group consisting of $C_{12}$-$C_{22}$ alkyl and $C_{12}$-$C_{22}$ alkenyl,

a salt or a stereoisomer thereof.

**9.** The chelating agent derivative of any of claims 1 to 8, further including, a paramagnetic metal ion or a radionuclide metal chelated with the chelating moiety.

**10.** A composition comprising a lipophilic nanoparticle or microparticle and a chelating agent derivative according to any of claims 1 to 9.

**11.** The composition of claim 10, wherein said lipophilic nanoparticle or microparticle further comprises a targeting agent or a biologically active agent.

**12.** An imaging method for imaging an object including a chelating agent derivative according to any of claims 1 to 9, or a composition according to any of claims 10 to 11, at a location to be imaged.

**13.** A method of manufacturing a chelating agent derivative according to any of the claims 1 to 9, comprising a step of reacting a phosphoethanolamine lipid or a derivative thereof with a chelating moiety having at least one carboxylic acid group.

**14.** An imaging system comprising:

- a chelating agent derivative according to any of claims 1 to 9, or a composition according to any of claims 10 to 11, and
- equipment for performing imaging of an object and adapted to detect said chelating agent derivative or composition.

**15.** Use of a chelating agent derivative according to any of claims 1 to 9, or a composition according to any of claims 10 to 11, in an imaging method.

**16.** A medical image created with a chelating agent derivative according to any of claims 1 to 9, or a composition according to any of claims 10 to 11.

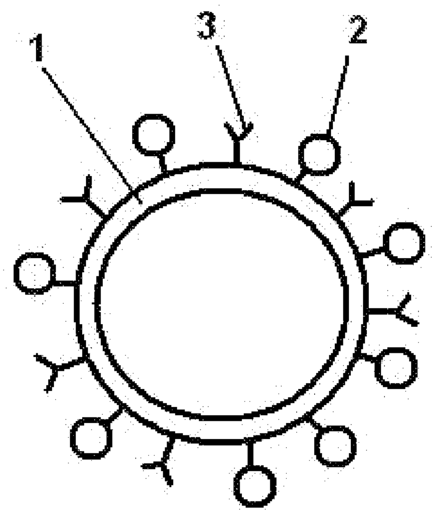


FIG. 1

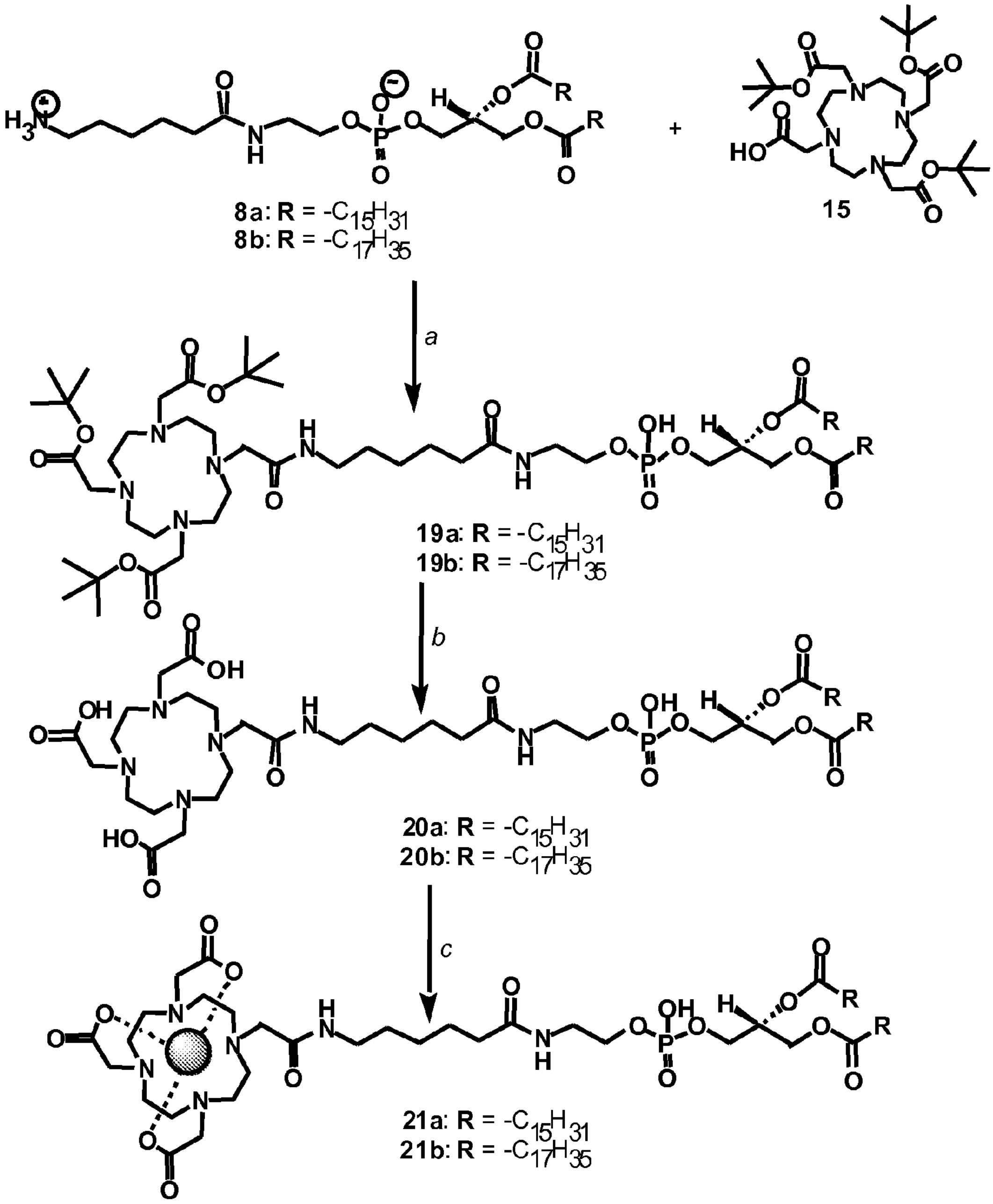


**8a**: **R** = -$C_{15}H_{31}$
**8b**: **R** = -$C_{17}H_{35}$

**15**

**19a**: **R** = -$C_{15}H_{31}$
**19b**: **R** = -$C_{17}H_{35}$

**20a**: **R** = -$C_{15}H_{31}$
**20b**: **R** = -$C_{17}H_{35}$

**21a**: **R** = -$C_{15}H_{31}$
**21b**: **R** = -$C_{17}H_{35}$

# FIG. 2

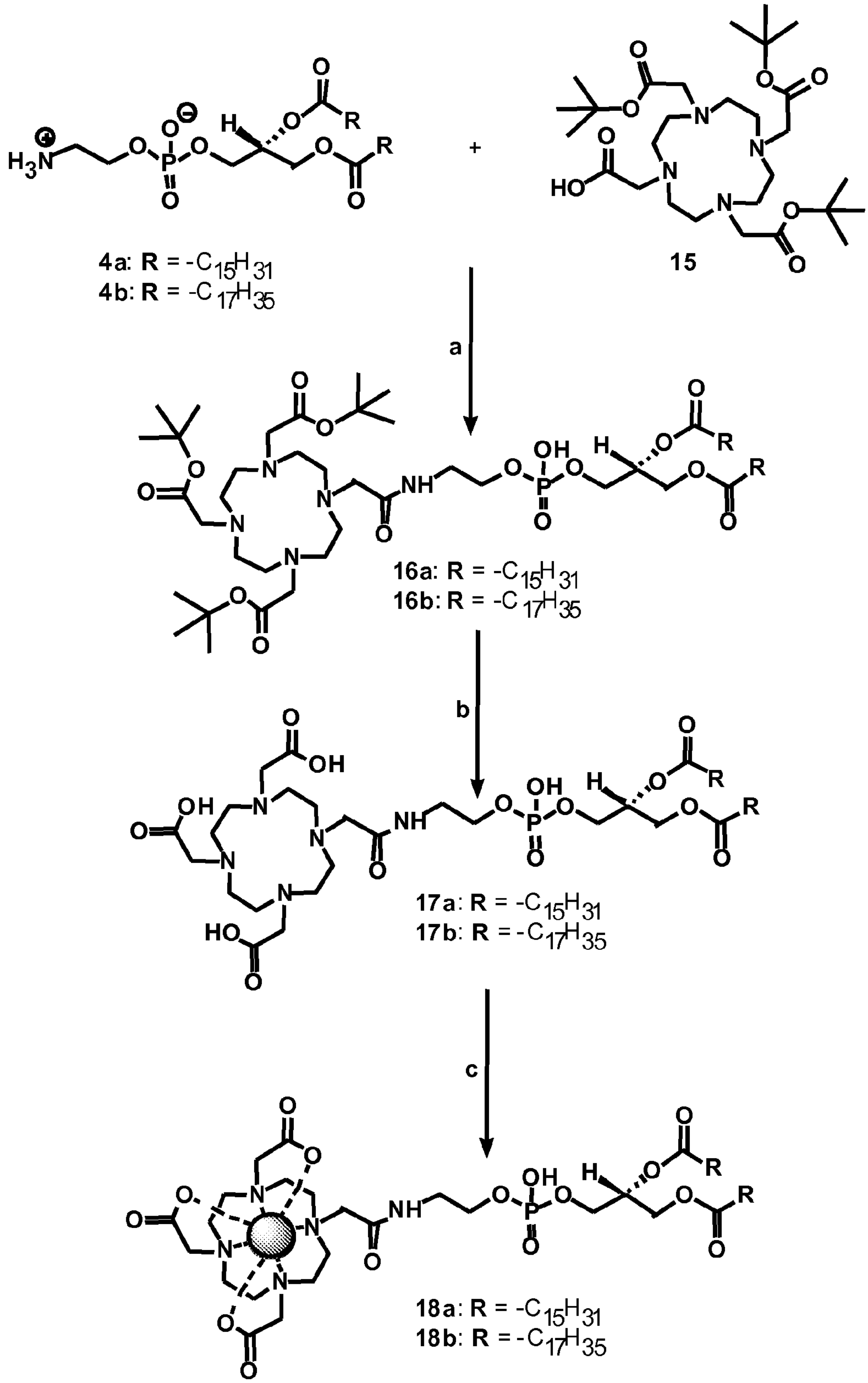


**4a**: **R** = $-C_{15}H_{31}$
**4b**: **R** = $-C_{17}H_{35}$

**15**

**16a**: **R** = $-C_{15}H_{31}$
**16b**: **R** = $-C_{17}H_{35}$

**17a**: **R** = $-C_{15}H_{31}$
**17b**: **R** = $-C_{17}H_{35}$

**18a**: **R** = $-C_{15}H_{31}$
**18b**: **R** = $-C_{17}H_{35}$

# Fig. 3

European Patent Office

# EUROPEAN SEARCH REPORT

Application Number
EP 07 12 2630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | WO 2006/032705 A (GUERBET SA [FR]; PORT MARC [FR]) 30 March 2006 (2006-03-30)<br>* examples 2,3 * | 1,6,7, 9-16<br>2-5,8 | INV. A61K49/08 |
| X<br><br>Y | WO 2006/100305 A (GUERBET SA [FR]; PORT MARC [FR]) 28 September 2006 (2006-09-28)<br>* examples 5,8,21 * | 1,6,7, 9-16<br>2-5,8 | |
| X<br><br><br>Y | WO 2004/067483 A (BARNES JEWISH HOSPITAL [US]; LANZA GREGORY M [US]; WICKLINE SAMUEL A [) 12 August 2004 (2004-08-12)<br>* claim 1; compound 4 * | 1,6,7, 9-16<br><br>2-5,8 | |
| X<br><br><br>Y | WO 2007/115115 A (KEREOS INC [US]; LANZA GREGORY M [US]; WICKLINE SAMUEL A [US]; KIEFER) 11 October 2007 (2007-10-11)<br>* figure 6; compounds 9-11 * | 1,6,7, 9-16<br><br>2-5,8 | |
| X<br><br>Y | URIZZI P ET AL: "EDTA and DTPA Analogues of Dipalmitoylphosphatidylethanolamine as Lipophilic Chelating Agents for Metal Labeling of LDL"<br>TETRAHEDRON LETTERS, ELSEVIER, AMSTERDAM, NL,<br>vol. 37, no. 27, 1 July 1996 (1996-07-01), pages 4685-4688, XP004028992<br>ISSN: 0040-4039<br>* figure 1 * | 1,6,7, 9-16<br><br>2-5,8 | TECHNICAL FIELDS SEARCHED (IPC)<br>A61K |
| X<br><br>Y | ALHAIQUE FRANCO ET AL: "Solvent 1H NMRD study of biotinylated paramagnetic liposomes containing Gd-bis-SDA-DTPA or Gd-DMPE-DTPA"<br>INORGANICA CHIMICA ACTA, X, XX,<br>vol. 331, 2002, pages 151-157, XP002428466<br>ISSN: 0020-1693<br>* figure 1 * | 1,6,7, 9-16<br><br>2-5,8 | |

-/--

The present search report has been drawn up for all claims

3

| Place of search | Date of completion of the search | Examiner |
|---|---|---|
| The Hague | 13 March 2008 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 07 12 2630

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | GRANT C W M ET AL: "A LIPOSOMAL MRI CONTRAST AGENT: PHOSPHATIDYLETHANOLAMINE-DTPA" MAGNETIC RESONANCE IN MEDICINE, ACADEMIC PRESS, DULUTH, MN, US, vol. 11, no. 2, 1 August 1989 (1989-08-01), pages 236-243, XP000054847 ISSN: 0740-3194<br>* compound B *<br>----- | 1,6,7, 9-16<br>2-5,8 | |
| X<br>Y | WINTER P ET AL: "Improved paramagnetic chelate for molecular imaging with MRI" JOURNAL OF MAGNETISM AND MAGNETIC MATERIALS, ELSEVIER SCIENCE PUBLISHERS, AMSTERDAM, NL, vol. 293, no. 1, May 2005 (2005-05), pages 540-545, XP004883029 ISSN: 0304-8853<br>* compounds 3,4 *<br>----- | 1,6,7, 9-16<br>2-5,8 | |
| X<br>Y | WO 2005/070400 A (SINAI SCHOOL MEDICINE [US]; FISHER EDWARD A [US]; FAYAD ZAHI A [US]; W) 4 August 2005 (2005-08-04)<br>* claims 14-16,68,76,77; figure 3 *<br>----- | 1,6,7, 9-16<br>2-5,8 | TECHNICAL FIELDS SEARCHED (IPC) |
| D,Y | WO 2005/122891 A (KEREOS INC [US]; RILEY DENNIS PATRICK [US]; MCGHEE WILLIAM D [US]) 29 December 2005 (2005-12-29)<br>* claims 1,18,19; compound 17 *<br>----- | 2-5,8 | |
| Y | US 2003/129579 A1 (BORNHOP DARRYL J [US] ET AL) 10 July 2003 (2003-07-10)<br>* compound 155 *<br>-----<br>-/-- | 2-5,8 | |

The present search report has been drawn up for all claims

3

| Place of search | Date of completion of the search | Examiner |
|---|---|---|
| The Hague | 13 March 2008 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 07 12 2630

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| E | WO 2007/141767 A (KONINKL PHILIPS ELECTRONICS NV [NL]; LAMERICHS RUDOLF M J N [NL]; WEGH) 13 December 2007 (2007-12-13) * page 19, line 28 - line 30; figure 2 * ----- | 1-16 | |
| | | | TECHNICAL FIELDS SEARCHED (IPC) |

The present search report has been drawn up for all claims

3

| Place of search | Date of completion of the search | Examiner |
|---|---|---|
| The Hague | 13 March 2008 | Schleifenbaum, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 07 12 2630

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2008

| Patent document cited in search report | | Publication date | Patent family member(s) | | | Publication date |
|---|---|---|---|---|---|---|
| WO 2006032705 | A | 30-03-2006 | EP | 1793868 | A2 | 13-06-2007 |
| | | | US | 2007292354 | A1 | 20-12-2007 |
| WO 2006100305 | A | 28-09-2006 | EP | 1885721 | A2 | 13-02-2008 |
| | | | FR | 2883562 | A1 | 29-09-2006 |
| WO 2004067483 | A | 12-08-2004 | AU | 2004207824 | A1 | 12-08-2004 |
| | | | CA | 2514201 | A1 | 12-08-2004 |
| | | | EP | 1594550 | A2 | 16-11-2005 |
| | | | JP | 2006516993 | T | 13-07-2006 |
| WO 2007115115 | A | 11-10-2007 | NONE | | | |
| WO 2005070400 | A | 04-08-2005 | EP | 1718282 | A1 | 08-11-2006 |
| WO 2005122891 | A | 29-12-2005 | AU | 2005253962 | A1 | 29-12-2005 |
| | | | CA | 2569461 | A1 | 29-12-2005 |
| | | | EP | 1768558 | A1 | 04-04-2007 |
| | | | JP | 2008502726 | T | 31-01-2008 |
| | | | US | 2006008417 | A1 | 12-01-2006 |
| US 2003129579 | A1 | 10-07-2003 | NONE | | | |
| WO 2007141767 | A | 13-12-2007 | NONE | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060008417 A


### Non-patent literature cited in the description


- **HEPPELER, A. et al.** *Chemistry-A European Journal,* 1999, vol. 5, 1974-1981
- **STRIJKERS, G. J. et al.** *Magnetic Resonance Materials in Physics Biology and Medicine,* 2005, vol. 18, 186-192